(19) Europäisches Patentamt / European Patent Office / Office européen des brevets

(11) **EP 1 722 374 B1**

(12) **EUROPEAN PATENT SPECIFICATION**

(45) Date of publication and mention
of the grant of the patent:
**14.08.2013 Bulletin 2013/33**

(51) Int Cl.:
*G01N 33/543* (2006.01)     *G01Q 60/42* (2010.01)
*B82Y 35/00* (2011.01)

(21) Application number: **05010477.7**

(22) Date of filing: **13.05.2005**

(54) **Method for determining the state of activation of a protein**

Verfahren zur Determinierung des Aktivationszustandes eines Proteins

Méthode de détérmination d'état d'activation d'une proteine

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**15.11.2006 Bulletin 2006/46**

(73) Proprietor: **nAmbition GmbH**
**01187 Dresden (DE)**

(72) Inventors:
• **Kedrov, Alexej**
**01099 Dresden (DE)**
• **Müller, Daniel J.**
**01099 Dresden (DE)**

(74) Representative: **Samson & Partner**
**Widenmayerstrasse 5**
**80538 München (DE)**

(56) References cited:
• FREDERIX P L T M ET AL: "Atomic force bio-analytics." CURRENT OPINION IN CHEMICAL BIOLOGY, vol. 7, no. 5, October 2003 (2003-10), pages 641-647, XP002340639 ISSN: 1367-5931
• KEDROV A ET AL: "Controlled Unfolding and Refolding of a Single Sodium-proton Antiporter using Atomic Force Microscopy" JOURNAL OF MOLECULAR BIOLOGY, LONDON, GB, vol. 340, no. 5, 23 July 2004 (2004-07-23), pages 1143-1152, XP004518124 ISSN: 0022-2836

• MUELLER DANIEL J ET AL: "Stability of bacteriorhodopsin alpha-helices and loops analyzed by single-molecule force spectroscopy." BIOPHYSICAL JOURNAL, vol. 83, no. 6, December 2002 (2002-12), pages 3578-3588, XP002340640 ISSN: 0006-3495
• IKAI ATSUSHI ET AL: "Toward mechanical manipulations of cell membranes and membrane proteins using an atomic force microscope: An invited review." CELL BIOCHEMISTRY AND BIOPHYSICS, vol. 39, no. 3, 2003, pages 257-277, XP008050999 ISSN: 1085-9195
• LECKBAND DEBORAH ET AL: "Measuring the forces that control protein interactions" ANNUAL REVIEW OF BIOPHYSICS AND BIOMOLECULAR STRUCTURE ANNUAL REVIEWS {A}, 4139 EL CAMINO WAY, PALO ALTO, CA, 94303-0139, USA SERIES : ANNUAL REVIEW OF BIOPHYSICS AND BIOMOLECULAR STRUCTURE (ISSN 1056-8700), 2000, pages 1-26, XP008051017 ISSN: 0-8243-1829-3
• RIEF M ET AL: "Unfolding forces of titin and fibronectin domains directly measured by AFM" ADVANCES IN EXPERIMENTAL MEDICINE AND BIOLOGY 2000 UNITED STATES, vol. 481, 2000, pages 129-141, XP008051015 ISSN: 0065-2598
• KELLERMAYER M S Z ET AL: "Folding-unfolding transitions in single titin molecules characterized with laser tweezers" SCIENCE, AMERICAN ASSOCIATION FOR THE ADVANCEMENT OF SCIENCE,, US, vol. 276, 16 May 1997 (1997-05-16), pages 1112-1116, XP002118923 ISSN: 0036-8075

EP 1 722 374 B1

## Description

The present invention relates to a method for determining the state of activation of a (poly)peptide, comprising the steps of: (a) immobilizing a (poly)peptide on a carrier; (b) attaching the (poly)peptide immobilized on the carrier to a force measuring device of a force spectroscope; (c) applying a pulling force to the (poly)peptide and measuring the force required for stretching and/or unfolding of the (poly)peptide attached to the carrier, wherein the measurements are carried out (i) prior to and (ii) after treatment with a known modulator of said (poly) peptide; (d) comparing the force spectra of the (poly)peptide prior to and after treatment with the modulator; and (e) concluding from a difference of the force spectra of step (d) on the state of activation of the (poly)peptide. Furthermore, the present invention relates to a method for determining whether a compound is an activator of (poly)peptide function, comprising the steps of (a) immobilizing a (poly)peptide on a carrier; (b) attaching the (poly)peptide to a force measuring device of a force spectroscope; (c) applying a pulling force to the (poly)peptide and measuring the force required for stretching and/or unfolding of the (poly)peptide attached to the carrier, wherein separate measurements are carried out in the presence of a test and a reference buffer, wherein the test buffer comprises a compound suspected of being an activator of the function of said (poly)peptide and wherein the reference buffer comprises (i) a compound known to have no effect on the activity of said (poly)peptide or (ii) a compound known to be an activator of said (poly)peptide; (d) comparing the force spectra of the (poly)peptide measured in the presence of the test and the reference buffer; and (e) concluding from a difference of the force spectra of step (d) whether the compound suspected of being an activator of said (poly)peptide is an activator of the (poly)peptide. Finally the present invention relates to a method for determining whether a compound is an inhibitor of (poly)peptide function.

For apparent reasons it is of prime interest for the pharmaceutical industry to be able to study in detail the state of activation of proteins which are the targets of their drugs. Atomic force-spectroscopy (AFM) has recently been used to gain some insight into the molecular response upon treatment of the target proteins with specific drugs. However, these experiments did not allow to detect and to locate the molecular interactions activating these proteins. Since more than a decade the tip of the AFM cantilever has served as a nano-tweezer, enabling to manipulate biological objects at the molecular scale [29,38,57,71,111]. The outstanding positioning precision (= 0.1 nm) and force sensitivity (= 5 pN) of the AFM has made even the most delicate single-molecule unfolding experiments using force spectroscopy possible. In these experiments, the force applied to a single protein plays the role of a denaturant leading to complete unfolding of its three-dimensional structure. In their initial studies, Rief and co-workers applied single-molecule force spectroscopy to the giant muscle protein titin, which consists of repeats of globular immunoglobulin and fibronectin domains [111,113]. The continuous extension of the protein resulted in the subsequent unfolding of the globular domains allowing the unfolding force and pathway of each domain to be detected [79,113,149].

In contrast to many experiments performed on water-soluble proteins, the application of single-molecule force spectroscopy to membrane proteins [93,103] yielded surprisingly detailed insights into the inter- and intramolecular interactions stabilizing their three-dimensional structure. This has been demonstrated on membrane proteins like BR [59,98], halorhodopsin [18] from *Halobacterium salinarum,* human aquaporin-1 [85], and the $Na^+/H^+$ antiporter NhaA from Escherichia coli [64]. To select a membrane protein for a force spectroscopy experiment the protein containing membrane was first imaged at sub-nanometer resolution. Then AFM tip and the selected protein are brought into contact. Applying a force of 300 - 1000 pN resuts in binding of one terminal end to the tip either via a covalent bond [103] or enforced non-specific adsorption [98]. Withdrawing the tip from the membrane stretches the terminus of the protein and causes the cantilever to deflect. Upon further separating the tip and surface, the force pulling on the protein steadily increases. As soon as the force exceeds the stability of the protein it induces the sequential unfolding of its three-dimensional structure. Recording the force against tip-surface separation yields a force-distance spectrum characteristic of the unfolding of a single protein. The presence of several distinct events in the force spectrum indicates that secondary structure elements of membrane proteins unfold in well-defined sequences. As their characteristic saw-tooth pattern stems from the extension of already unfolded polypeptide elements, the unfolding spectra are readily analyzed with the wormlike chain (WLC) model.

Single-molecule force spectroscopy [14,73,150] provide novel approaches to characterize water-soluble and membrane proteins under variable physiological environments [22,45,46]. In all measurements the proteins were exposed to buffer solution at ambient temperatures. It was shown in several examples, that single-molecule force spectroscopy enables to detect inter- and intramolecular interactions within and between proteins [65,79,103,113]. Such experiments not only enabled to detect the stability of membrane proteins [98], but also to probe their energy landscape [60] and refolding kinetics [64]. Single potential barriers confine structurally stable segments that may be represented by transmembrane alpha-helices, polypeptide loops or fragments hereof. These structural segments are established by collective interactions of several amino acids. Once the externally applied force overcomes the stability of these segments they unfold spontaneously. The first experiments allowed investigating how environmental variations such as the oligomeric assembly [115], temperature changes [59], point mutations [98], or pH variations [64] influenced the stability of these structural segments and thereby the unfolding pathways of the protein. Comparing structurally stable segments established within two different membrane proteins having almost identical structures allowed to gain insights into the origin of these interactions [18]. Recently, it has become possible to observe the refolding of secondary structure elements into the final protein and to estimate their folding kinetics from single-molecule experiments [64].

Until now, force-spectroscopy experiments performed on single proteins did not allow to detect and to locate molecular interactions that activate a protein. Moreover, it could not be shown at which location a ligand binds to the protein and thereby activates the protein function. Monitoring the state of activation of a protein would, however, permit to test the effect of test compounds on the functional state of proteins and, thus, allow to more effectively screen for physiologically active drug candidates.

Müller, D. et al., Biophysical Journal , Vol. 83, 3578-3588 (2002), disclose single-molecule force spectra of bacteriorhodopsin measured at different pH values in a buffer containing KCl. Frederix PLTM et al., Curr. Op. Chem. Biol., vol. 7, 641-647 (2003) refer to the article of Müller.

Thus, the technical problem underlying the present invention was to provide methods for determining the functional state of proteins.

The solution to this technical problem is achieved by providing the embodiments characterized in the claims.

Accordingly, the present invention relates to a method for determining the state of activation of a (poly) peptide, comprising the steps of: (a) immobilizing a (poly) peptide on a carrier; (b) attaching the (poly)peptide immobilized on the carrier to a force measuring device of a force spectroscope; (c) applying a pulling force to the (poly)peptide and measuring the force required for stretching and/or unfolding of the (poly)peptide attached to the carrier, wherein the measurements are carried out (i) prior to and (ii) after treatment with a known modulator of said (poly)peptide; (d) comparing the force spectra of the (poly)peptide prior to and after treatment with the modulator; and (e) concluding from a difference of the force spectra of step (d) on the state of activation of the (poly)peptide.

The term the "known modulator" as used herein refers to a compound known to have a particular effect on the state of activation or function of a given (poly) peptide. The known modulator may be for example a known activator, inhibitor, co-factor, substrate or ligand of said (poly)peptide. As explained herein, such a compound - when interacting with the (poly)peptide - will also have an effect on the molecular interactions within the (poly)peptide, stabilizing or destabilizing secondary structure elements (such as alpha-helices or beta-sheets) which will be reflected in the force spectra of the (poly)peptide. In particular cases such a modulator only affect the conformation of the (poly)peptide without having an obvious effect on the activity (of the (poly)peptide (e.g. the conversion rate of an enzyme). In these cases, the modulator may be a cofactor affecting the binding of additional cofactors of the (poly)peptide which in turn may have an effect on the state of activation of the (poly)peptide.

The term "(poly)peptide" refers to peptides, polypeptides and proteins. As used herein, peptides have up to approximately 30 residues, polypeptides between 31 and approximately 50 residues, whereas proteins have at least 51 residues. It is, however, apparent that some small proteins may be made up of a lower number of amino acid residues such as e.g. 35 or 40. The term "(poly)peptide" refers to wild-type (poly)peptide but also to mutants of particular (poly)peptides. It is important to note that the present invention is particularly useful for screening such mutants in order to find out whether or not a particular mutation has an effect on the state of activation of the (poly)peptide. To this end, the force spectra of wild-type and mutant (poly)peptides obtained from measurements in the presence and absence of a known modulator can be compared. If a particular mutation has an effect on e.g. binding of a known activator or if the mutation results in blocking of a conformational change required for activation of the (poly)peptide, this would result in a different force spectrum. The difference would thus indicate to the skilled person that the (poly) peptide mutant no longer is capable of being activated by said known activator.

Immobilizing a (poly)peptide on a carrier can be achieved by covalently coupling the polypeptides to the carrier or by using non-covalent interactions. Non-covalent interactions may e.g. be mediated by antibodies or other biological molecules such as avidin, biotin, histidine tags, collagen binding domains, streptavidin, fibronectin, which are capable of specifically attaching the (poly)peptides. Other non-covalent interactions resulting in immobilization of the (poly)peptides may be based on hydrophobic, hydrophilic, electrostatic or ionic interactions of the (poly)peptides with the carrier.

The term "force measuring device of a force spectroscope" refers to a cantilever at which the (poly) peptide is attached to. Alternative "force measuring devices" may for example use a dithering tip or a bead or a vesicle to detect molecular forces.

The method of the present invention can be applied to proteins which can exist in at least two states, one of which is an active state or a state which activity has changed in which the (poly)peptide exerts one of its functions. Accordingly, the term "determining the state of activation" means finding out whether a (poly)peptide is in its active state and, optionally, if so, in which state of activation (see below).

The inactive state is the state in which the protein is e.g. switched off. In case the (poly)peptide is an enzyme, this state may be the state with a reduced or blocked catalytic activity. The inactive state of a (poly)peptide may, however, also be a denatured state of a (poly)peptide. The active state may be also presented by the state at which the protein exhibits no functional activity. In case of an ion channel, this may be the state at which the channel is closed.

While some proteins switch between an active and an inactive state, other proteins have multiple states of activation. Allosteric proteins are one example of such proteins which can switch, e.g. upon binding of specific ligands, between a number of states of activation (e.g. states A ->B->C->D ...etc.). Each of these states may have difference ligand binding constants. Importantly, the switch between different states of activation is generally accompanied by a conformational rearrangement within the three-dimensional organization of the protein. This structural rearrangement may be limited to only few amino acid residues often located near the active site of the protein, it may affect isolated regions within the secondary structure of the protein (such as an entire alpha helix or beta sheet arrangement) or affect entire subunits of the protein. Such conformational rearrangements, as discussed above, have implications on the internal stability of the proteins, even if only very few amino acid residues are affected. The present invention's methods exploit the idea that each state of activation comes along with a characteristic set of molecular interactions, which on the one hand hold together the three-dimensional organization of the protein and on the other hand drive the protein function. For example, binding of a ligand in the active center of a protein may stabilize the arrangement of the amino acid residues involved in formation of the binding pocket. Accordingly, when such a protein is analyzed by the methods of the present invention, additional pulling force is required for unfolding of the structural elements involved in the formation of the binding pocket. This additional force will be reflected in a change of the force spectrum, in particular in the position of the amino acid residues located near the ligand binding site. Accordingly, the methods of the present invention allow a differentiation between different functional states of proteins. The assignment a particular state of activation of a (poly)peptide is possible because the methods of the present invention rely on a comparison of the force spectra with a "reference state". This reference state is the state of the (poly)peptide after treatment with a "reference modulator", i.e. a modulator with a known effect on the (poly)peptide. A given (poly)peptide is, for example, in its active state when treated under suitable conditions with a known activator. Alternatively, the (poly)peptide is in its inactive state when treated with a compound known to block activation of the (poly)peptide.

Applying the present invention's method, the inventors have been able to generate force curves containing detailed information about strength and location of molecular interactions established within NhaA, an *Escherichia coli* antiporter specifically involved in exchange of $Na^+$ ions for $H^+$, allowing the cell to adapt to high environmental salinity and to grow at alkaline pH (Padan et al., 2001). Moreover, these force curves allow to determine the functional state of the NhaA protein at particular environmental conditions. As shown in detail in the Examples, while molecular interactions stabilizing secondary structure elements remained unaffected on switching NhaA into its functional (active) state, those being assigned to the $Na^+$ binding site changed dramatically. As illustrated by this example, the direct observation of molecular interactions provides novel insights into activation mechanisms of proteins.

In a preferred embodiment of the present invention, the modulator is a known activator, inhibitor, cofactor, substrate or ligand of said (poly)peptide. In fact, this method of the present invention may be performed with any compound with a known effect on the (poly)peptide function. The only constraint, as explained above, is the fact that an assignment to a particular state of activation must be possible.

The present invention also relates to a method for determining the state of activation of a (poly)peptide, comprising the steps of: (a) immobilizing a (poly)peptide on a carrier; (b) attaching the (poly)peptide immobilized on the carrier to a force measuring device of a force spectroscope; (c) applying a pulling force to the (poly)peptide and measuring the force required for stretching and/or unfolding of the (poly)peptide attached to the carrier; (d) comparing the force spectra of the (poly)peptide of (c) with a control representing an inactive or a known state of activation of the (poly)peptide ; and (e) concluding from a difference, if any, of the force spectra of step (d) on the state of activation of the (poly)peptide.

The present invention also relates to a method for determining whether a compound is an activator of (poly)peptide function, comprising the steps of: (a) immobilizing a (poly)peptide on a carrier; (b) attaching the (poly)peptide to a force measuring device of a force spectroscope; (c) applying a pulling force to the (poly)peptide and measuring the force required for stretching and/or unfolding of the (poly)peptide attached to the carrier, wherein separate measurements are carried out in the presence of a test and a reference buffer, wherein the test buffer comprises a compound suspected of being an activator of the function of said (poly)peptide and wherein the reference buffer comprises (i) a compound known to have no effect on the activity of said (poly)peptide or (ii) a compound known to be an activator of said (poly)peptide; (d) comparing the force spectra of the (poly)peptide measured in the presence of the test and the reference buffer; and (e) concluding from a difference of the force spectra of step (d) whether the compound suspected of being an activator of said (poly)peptide is an activator of the (poly)peptide.

The term "compound known to have no effect on the activity of said (poly)peptide" refers to a compound which does not affect the function of the (poly)peptide. Such a compound does usually not have an effect on the force spectrum of the (poly)peptide.

The term "activator" refers to a compound having a positive effect on the activity of the (poly)peptide. Hence, such a compound may e.g. increase the catalytic activity of an enzyme. The activator may be e.g. a proteinaceous compound, a peptide or a chemical entity such as a small molecule.

Depending on whether reference buffer (i) or (ii) is used in this method, the comparison of the force spectra will yield different results:

When using reference buffer (i):

(a) the observation of no difference between the force spectra generated by the test and the reference buffer of step (d) will allow to conclude that the compound contained in the test buffer did not interact with the (poly)peptide and is no activator of said (poly)peptide.
(b) the observation of a difference between the force spectra generated by the test and the reference buffer of step (d) will allow to conclude that the compound contained in the test buffer interacted with the (poly)peptide and is an activator of said (poly)peptide.

When using reference buffer (ii):

(a) the observation of no difference between the force spectra generated by the test and the reference buffer of step (d) will allow to conclude that the compound contained in the test buffer interacted with the (poly)peptide and is an activator of said (poly)peptide.
(b) the observation of a difference between the force spectra generated by the test and the reference buffer of step (d) will allow to conclude that the compound contained in the test buffer did not interact with the (poly)peptide and is no activator of said (poly)peptide.

As shown in the Examples, from comparing the force spectra of the test and the reference buffer it is also possible to determine the amino acid residues of the (poly)peptide that interacted with the compound contained in the reference buffer.

The above described screening assay represents an adaptation of the first assay described in the present invention and allows to screen compounds with respect to their capability to modulate the state of activation of a given (poly)peptide. According to this method, the state of activation of a (poly)peptide is monitored in the presence and absence of a potential activator of said (poly)peptide. Binding of an activating compound will induce a change of the force spectra which is indicative of the compound's capability to modulate the function of the (poly)peptide.

It is important to note that some proteins can bind specific activators as well as other specific cofactors required for optimal protein function. In fact, the binding constant of an activator of a protein may be affected from binding of a cofactor. In such cases, potential activators may also be screened after treatment and/or in the presence of a known specific cofactor.

The present invention also relates to a method for determining whether a compound is an inhibitor of (poly)peptide function, comprising the steps of: (a) immobilizing a (poly)peptide on a carrier; (b) attaching the (poly)peptide to a force measuring device of a force spectroscope; (c) applying a pulling force to the (poly)peptide and measuring the force required for stretching and/or unfolding of the (poly)peptide attached to the carrier, wherein separate measurements are carried out in the presence of a test and a reference buffer, wherein the test buffer comprises a compound suspected of being an inhibitor of the function of said (poly)peptide and wherein the reference buffer comprises (i) a compound known to have no effect on the activity of said (poly)peptide; (ii) a compound known to be an activator of said (poly)peptide; (iii) a compound known to be an inhibitor of said (poly)peptide; (d) comparing the force spectra of the (poly)peptide measured in the presence of the test and the reference buffer; and (e) concluding from a difference of the force spectra of step (d) whether the compound suspected of being an inhibitor of said (poly)peptide is an inhibitor of the (poly)peptide.

The term "inhibitor" refers to a compound having a negative effect on the activity of the (poly)peptide. Hence, such a compound may e.g. reduce or block the catalytic activity of an enzyme. The inhibitor may be e.g. a proteinaceous compound, a peptide or a chemical entity such as a small molecule, it may be isolated from nature or be a synthetic compound.

When using reference buffer (i):

(a) the observation of no difference between the force spectra generated by the test and the reference buffer of step (d) will allow to conclude that the compound contained in the test buffer did not interact with the (poly)peptide and is no inhibitor of said (poly)peptide.
(b) the observation of a difference between the force spectra generated by the test and the reference buffer of step (d) will allow to conclude that the compound contained in the test buffer interacted with the (poly)peptide and is an inhibitor of said (poly)peptide.

When using reference buffer (ii):

(a) the observation of no difference between the force spectra generated by the test and the reference buffer of step (d) will allow to conclude that the compound contained in the test buffer interacted with the (poly)peptide and is an inhibitor of said (poly)peptide (b) the observation of a difference between the force spectra generated by the test and the reference buffer of step (d) will allow to conclude that the compound contained in the test buffer did not interact with the (poly)peptide and is no inhibitor of said (poly)peptide.

When using reference buffer (iii):

(a) the observation of no difference between the force spectra generated by the test and the reference buffer of step (d) will allow to conclude that the compound contained in the test buffer interacted with the (poly)peptide and is an inhibitor of said (poly)peptide (b) the observation of a difference between the force spectra generated by the test and the reference buffer of step (d) will allow to conclude that the compound contained in the test buffer did not interact with the (poly)peptide and is no inhibitor of said (poly)peptide.

As shown in the Examples, from comparing the force spectra of the test and the reference buffer it is also possible to determine the amino acid residues of the (poly)peptide that interacted with the compound contained in the reference buffer. The characteristic force spectroscopy spectra revealed unfolding NhaA contains sets of intensive force peaks. To assign the molecular interactions that were established within the protein each force peak of the force spectra is fitted using the worm-like chain (WLC) model. The fit revealed the length of the stretched polypeptide and allowed to assign polypeptide regions which formed molecular interactions. If a compound interacts with a certain region of the (poly)peptide this will change molecular interactions which are then measured by the force spectroscopy experiments. The additional or changed force peak caused due to the compound interaction will be located and quantified using the above procedure.

The above described screening assay represents an adaptation of the first assay described in the present invention and allows to screen compounds with respect to their capability to inhibit the activity of a given (poly)peptide. According to this method, the state of activation of the (poly)peptide is monitored in the presence and absence of a potential inhibitor of said (poly)peptide. Binding of an inhibiting compound will induce a change of the force spectra which is indicative of the compound's capability to modulate the function of the (poly)peptide.

It is important to note that some proteins can bind specific activators as well as specific inhibitors of their protein function. In fact, some proteins can bind a number of additional factors, all of which may affect substrate binding and/or binding of the individual factors.

Hence, the binding constant of a second binding partner of the protein (e.g. an inhibitor) may be affected from binding of a first modulator (e.g. an activator). In such cases, potential inhibitors may also be screened after treatment and/or in the presence of other known binding partners of said protein, such as specific activators or specific cofactors of the protein.

In a preferred embodiment of the present invention, the carrier is (a) a biological carrier selected from the group consisting of membrane, vesicle, cell, interface such as cell membranes, membrane of vesicles, lipid membranes, extracellular matrix and compound hereof; biological structure such as lipids, collagen, actin, microtubules, cytoskeleton, collagen, actin, microtubules, protein and protein complexes, aggregates such as amyloid fibers and plaques, bone; protein, nucleic acid molecule, fibril, fibre, and biological scaffold (extracellular matrix, tissues, cells) or (b) a non-biological carrier selected from the group consisting of solid state material such as mica, graphite, silicium, gold, gallium arsenide; polymer such as Polyethylene, polypropylene, polystyrol, acrylate, collagen; synthetic membrane such as co-block polymers, or membrane assembled from synthetic lipids; and synthetic scaffold such as peptide hydrogels, hydrogels, polymer nanofibers, pegpamam star polymers.

In another preferred embodiment of the present invention, the (poly)peptide is arranged as a three-dimensional crystal, two-dimensional crystal, bound to a biological or non-biological surface (e.g. solid state material, polymer, synthetic membrane, synthetic scaffold), attached to a membrane or peptide, attached to any biological or synthetic molecule (e.g. amines, antibiotics, hormones, enigmols, supramolecular cylinders $[Fe_2L_3]^{4+}$), or incorporated into a membrane. The (poly)peptide may be attached or bound covalently (e.g. using C- or S-bonds) or non-covalently (e.g. via electrostatic, hydrophilic, hydrophobic, ionic, electrostatic, magnetic or van der Waals interactions).

In another preferred embodiment of the present invention, the (poly)peptide is embedded in a lipid bilayer. The lipid bilayer may be derived (i.e. obtainable) from a biological membrane. Alternatively, the lipid bilayer may be a synthetic lipid bilayer. Synthetic means that that it is generated from its constituents in vitro. To this end, molecules such as phospholipids, sphingolipids, glycolipids, cholosterol and a other biological or synthetic molecules may be mixed in the presence or after addition of the (poly)peptide.

In yet another preferred embodiment of the present invention, the measuring device is selected from (a) tip of a force spectroscope, (b) a magnetic tweezer, (c) an optical tweezer, (d) a protein or protein complex, (e) by any other biological or synthetic molecule such as a peptide, protein, protein complex, lipid, nucleic acid, (f) a surfaces of a force apparatus such as mica, graphite, silicium, gold, gallium arsenide, (g) a membrane such as a cell membrane, vesicle, lipid bilayer, lipid monolayer, co-block polymer, surface layer of a bacteria or vertebrate cell, and (h) the probe of a scanning probe microscope or a scanning probe spectroscope.

In another preferred embodiment of the present invention, the (poly)peptide is attached to the measuring device by non-covalent interactions. The term non-covalent interaction, as used throughout the specification means hydrophobic, hydrophilic or steric interactions or/and interactions by charge (e.g. van der Waals, ionic, electrostatic). To this end, the measuring device and the (poly)peptide are brought into contact. Applying a force of 300 to 1000 pN results in binding of one terminal end to the measuring device, either via a covalent bond or enforced non-specific adsorption.

In another preferred embodiment of the present invention, the pulling force applied from the tip of the force spectroscope is in the range of 1 to 500 pN. The maximum pulling force is determined by the strength of the molecular interaction established within the protein. The molecular interaction can also result from the binding of the compound.

In another preferred embodiment of the present invention, stretching and/or unfolding are measured by recording the unfolding and/or stretching resistance observed upon application of the pulling force from the tip of the force spectroscope to the (poly)peptide. It is to be noted that the force applied to a single protein plays the role of a denaturant leading from continuous extension of a protein to complete unfolding of its three-dimensional structure. The force required for withdrawing the measuring device attached to the (poly)peptide from the carrier represents the pulling force. As soon as this pulling force exceeds the stability of the protein, it induces the sequential unfolding of its three-dimensional structure which is recorded.

In another preferred embodiment of the present invention, said activator is an agonist. The term "agonist" refers to analogues of a native or synthetic ligand (for example a protein or a hormone) that binds to a specific receptor and triggers the receptor activity function. It does not matter whether the ligand is a natural or synthetic compound. Most important however, is that the binding of the ligand induces a biological reaction, such as the activation of a receptor, which then can activate a biological process. One example is given by the acetylcholine receptor, which opens the channel after binding of the antagonist acetylcholine. The term "antagonist" describes analogues that act as competitive inhibitors against agonist binding. They may also displace the agonist from the receptor and occupy the appropriate receptor. As a result the antagonist prevents receptor activation or changes the receptor function.

In another preferred embodiment of the present invention, said activator or inhibitor is selected from the group consisting of a protein specific ligand (for example a protein or a hormone), a synthetic compound such as a synthetic ligand, ligand, activator, agonist, antagonist; synthetic or substitutions of acetylcholine, nicotine, glutamate, dopamine, hydroxytryptamine, serotonin, pheromones and interleukin; a pharmaceutical compound such as a hormone, a toxin (e.g. acetylcholine, nicotine, glutamate, dopamine, hydroxytryptamine, serotonin, pheromones, interleukin, hormone toxin); a biochemical or biological compound such as a ligand, hormone, (poly)peptide, cholesterol, lipid, signaling molecule, acetylcholine, nicotine, glutamate, dopamine, hydroxytryptamine, serotonin, pheromones, interleukin; lipid, (poly)peptide, light, electrolyte, pH, voltage or current, being capable of inducing a change of the functional state (activity) of said (poly)peptide.

In a preferred embodiment of the present invention, more than one compound suspected of being an activator or inhibitor of the (poly)peptide function is tested.

In another preferred embodiment of the present invention, the measurement is performed in the presence of a compound which is an agonist or antagonist of said activator or inhibitor.

In a more preferred embodiment of the present invention, said compound is part of a compound library and wherein said method contains the additional step of screening said compound library for identifying a lead compound for drug development.

In another more preferred embodiment of the present invention, said screening is high-throughput screening carried out by multiple cantilevers and/or by multiple force spectroscopes and/or by fast-speed force spectroscopy.

In a preferred embodiment of the present invention, the (poly)peptide is a protein.

In a more preferred embodiment of the present invention, said protein is selected from the group consisting of (a) ligand-gated receptor, (b) G-protein coupled receptor, (c) ion channel, (d) water channel, (e) antiporter, (f) communication channel, (g) symporter, (h) porin, (i) ion gating channel, electrolyte gated channel and pore, ion pump, glutamate gated ion channel, ATP gated channel, (j) mechano-sensitive channel and (k) ATP synthase.

Ligand-gated receptors include for example nicotinic acetylcholine receptor, hydroxytryptamine serotonin receptor, GABA A receptor, GABA B receptor. G-protein coupled receptors include molecules such as rhodopsin, Vasopressin V2 receptor, Metabotropic glutamate receptor, Interleukin-8 receptor, Adrenergic receptor, Neuropeptide Y receptor, Dopamine 1 B receptor, Glycoprotein hormone receptor, Melanocortin receptor, Adenosine receptor, Pheromone A receptor. Ion channels include molecules such as Potassium channel, sodium channel, calcium channel, Slow voltage-gated potassium channel, NMDA receptor, P2X5 purinoceptor, Chloride channel CLC, Influenza virus matrix protein M2, Calsequestrin, Arsenical pump membrane protein, MscS and MscL mechanosensitive ion channels, Voltage-dependent calcium channel. Water channels include molecules such as aquaporin 1-10, MIP, GlpF. Pores include for example Channel forming colicins, ATP P2X receptor, Delta-endotoxin CytB, P2X purinoceptors, Mammalian defensin, Proteinase inhibitor I17, Haemolysin E. Antiporters include for example Sodium/proton antiporter, betaine/proton antiporter, Cadmium-transporting ATPase, Anion exchange protein, Sodium/calcium exchanger, Calcium/proton exchanger, Sodium/hydrogen exchanger, Multicomponent $K^+:H^+$ antiporter, $K^+$-dependent $Na^+/Ca^+$ exchanger, Multicomponent $Na^+:H^+$ antiporter, Sodium/hydrogen exchanger. Communication channels include for example Gap junctions, connexins. Symporters include for example LacY, Sodium: alanine symporter, $Na^+$ dependent nucleoside transporter, Glycine neurotransmitter transporter, Sodium/glutamate symporter, Pentulose kinase. Porins include for example OmpF porin, maltoporin. Additional (poly)peptide or proteins that may be analyzed using the methods of the present invention are ion gating channels, electrolyte gated channels and pores, ion pumps, glutamate gated ion channels, ATP gated channels or mechano sensitive channels such as MscS and MscL and ATP synthases such as F-, V-, or H-type.

In a preferred embodiment of the present invention, the pulling force is exerted by the measuring device. In this case the cantilever (if measuring device) is moved to stretch the protein.

In another preferred embodiment of the present invention, the pulling force is exerted by the carrier. In this case the carrier is moved to stretch the protein.

In another preferred embodiment of the present invention, the pulling force is exerted by the (poly)peptide. In this case the (poly)peptide exerts forces that deflect the cantilever. Positions of the cantilever and carrier are not changed in this case.

In another preferred embodiment of the present invention the pulling force is exerted by applying two or three of the above embodiments.

The figures show:

**Figure 1:**

Detecting pH and $Na^+$ dependence of molecular interactions established within NhaA. (A) Left, representative force-extension curve recorded upon mechanical unfolding a single NhaA molecule. Each force peak is fitted by the WLC model (red curves) with the numbers of stretched aa residues given. Right, secondary structure of NhaA mapped with stable structural segments detected (grey shaded) upon pulling the C-terminus. Grey gradients reflect uncertainties in determining segment positions. To determine merges of the segments on the opposite side to the AFM tip of the membrane or within, the membrane thickness of 4 nm was considered (Kedrov, 2004). In these cases corresponding contour lengths are given in brackets. (B-F), superimpositions of extension curves recorded upon single NhaA unfolding at pH 3.8 (B) and (C) 5.5 (inactive states) and (D) pH 7.7 (active state) at electrolyte concentrations of 150mM KCl and 50mM NaCl. The pH-dependent unfolding peak at 225aa is encircled. To prove the reversibility of the pH dependent change NhaA was incubated for 1h at pH 7.7 and unfolded at pH 3.8 (E). Significant restoration of the protein stability suggests the reversibility of molecular interactions. (F) Force-extension curves of NhaA recorded at pH 7.7 in absence of NaCl reduced the molecular interaction to that measured for the inactive state. 20 force-extension curves were superimposed for each figure. (G) Average unfolding forces of helical pairs. Forces and standard deviations are plotted for different pH values at 150mM KCl and 50mM NaCl. Pairs of neighboring helices tend to unfold cooperatively giving force peaks at 163aa (helices VII&VIII), 202aa (helices V&VI), 258aa (helices III&IV) and 328aa (helices I&II)(Kedrov, 2004).

**Figure 2:**

pH and $Na^+$ dependent molecular interactions established by at the active site of NhaA. Change in stability of helix V derived from single-molecule unfolding events. Distribution of unfolding forces of helix V (peak 225aa) in presence of $Na^+$ is given by histograms for pH 3.8 (A), 5.5 (B), and 7.7 (C). (D) Removal of $Na^+$ ions from the buffer solution reduced the molecular interaction to that measured for inactive NhaA (A and B). Approximately 70 single-molecule unfolding spectra were analyzed at each pH (see Materials and Methods). Distributions A and C, C and D are statistically different with significance of $p < 0.001$. Black bars on the left of the histograms represent unfolding events, where no peak was detected at 225aa.

**Figure 3:**

Characterizing molecular forces established at ligand binding site of NhaA. Strength (A) and frequency (B) of molecular interactions established at ligand binding site (C) increase upon changing pH from 5 to 6. Solid lines represent sigmoid fits of the data points. Dashed lines indicate pH values at which the mid-points of transitions were reached. (C) Primary and secondary structure of helix V. Aspartic acids (D163 and 164) of the $Na^+$ binding site were indicated. pH changes enable accessibility of $Na^+$ ions.

The examples illustrate the invention:

**Example 1:**

*NhaA remains fully folded over wide pH range*

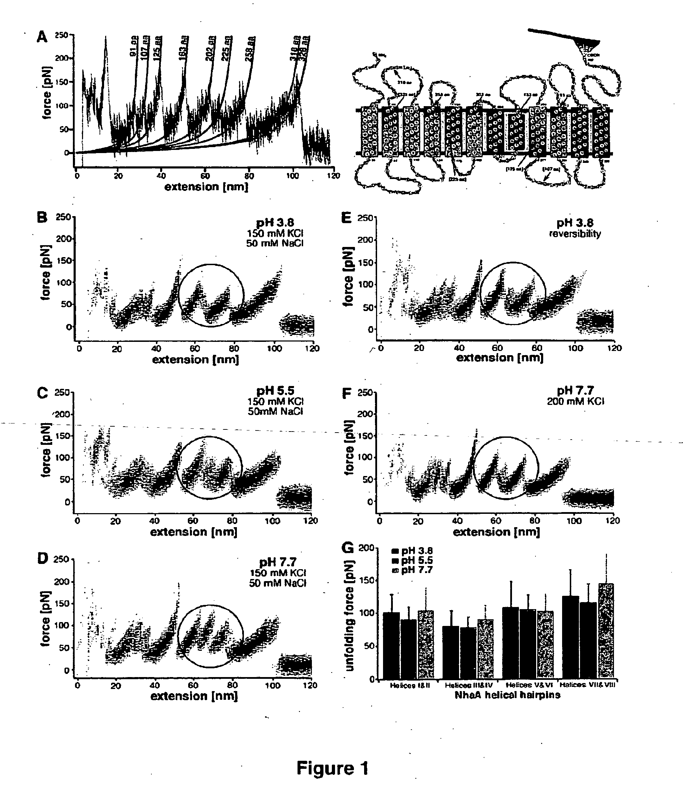

Figures 1B-D show superimpositions of force-extension curves recorded upon unfolding individual NhaA molecules at different pH values. They correspond to unfolding of inactive (pH 3.8 and 5.5) and fully active (pH 7.7) forms of NhaA (Taglicht et al., 1991). All superimpositions show the characteristic unfolding spectra of NhaA as reported (Kedrov, 2004) and reveal no additional unfolding events. The unfolding peaks detected allow locating structural segments, which formed an unfolding barrier (Figure 1A). Overcoming the critical force initiates cooperative unfolding of all aa within the structural segment, which established the stabilizing molecular interactions (Kedrov, 2004). These stable segments do not necessarily correlate to a single secondary structure element of the protein. For example, the force spectra showed unfolding of a transmembrane helix together with a polypeptide loop, or of two helices collectively establishing an unfolding barrier (Figure 1A). Each barrier then unfolds cooperatively upon mechanically pulling.

Average forces required to unfold helical pairs I&11, III&IV, V&VI and VII&VIII (Figure 1G) show that their stability is retained independent of the pH range of 3.8 to 7.7. Both, the unaffected stability and locations of molecular interactions stabilizing the structural domains of NhaA imply that they do not change upon protein activation. Hence, it can be concluded that the protein maintained its folded stable conformation in the experiments.

## Example 2:

*alpha-helix V establishes molecular interactions*

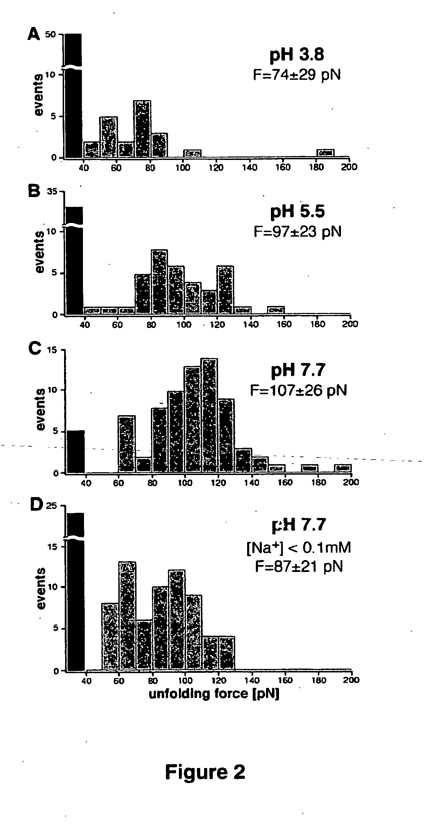

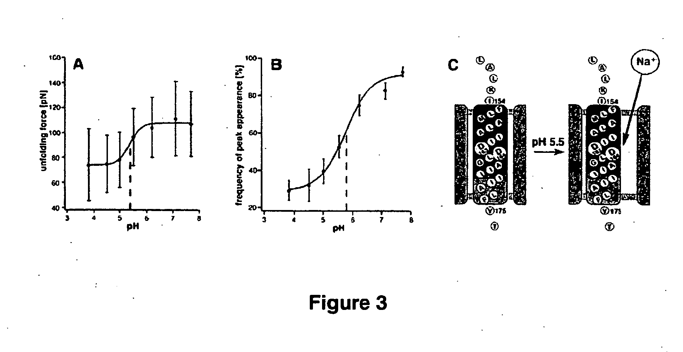

While the general profile of NhaA unfolding curves remained unchanged upon pH variation, the molecular interactions establishing the force peak 225aa increased significantly (Figure 1B-D; encircled areas). On the basis of the primary and presumed secondary structure of NhaA (Rothman et al., 1996), we recently showed that the corresponding unfolding barrier was located at the middle of transmembrane helix V (Figure 1A) (Kedrov, 2004). Overcoming the molecular interactions stabilizing this structural region by an externally applied force induces unfolding of the cytoplasmic half of helix V. On raising the pH from 3.8 to 7.7, the average force required to overcome the molecular interactions increased from $74\pm29$ pN to $107\pm26$ pN (average $\pm$ SD). Thus, in fully active NhaA the molecular interactions established within this region reached the strength typically measured for unfolding of a helical pair (Figure 1 G, 3A). Simultaneously, the frequency of peak detection increased from 31 to 94% (Figure 3B). Detailed insights into the kinetics of this local stabilization were achieved analyzing single-molecule unfolding events (Figure 2A-C). The histograms of the unfolding forces distribution clearly show an increased frequency of the 225aa peak while approaching functional pH values. The increased stability of this region shifted the force distribution gradually to ~100-120 pN. Reversing the pH from 7.7 to 3.8 restored initial molecular interactions (Figure 1E) as the mean unfolding force reduced to values ($78\pm30$ pN) similar to that detected for the inactive form of NhaA ($74\pm29$ pN).

## Example 3:

*pH dependent molecular interactions co-localize with ligand-binding site*

Several studies on NhaA imply that the negatively charged aspartic acid residues 163 and 164 are involved in the $Na^+$-binding site located in the center of transmembrane helix V. Substitution of these residues with cysteines or asparagines dramatically reduce the cation transport activity of NhaA (Inoue et al., 1995; Padan et al., 2001). It has been shown for different membrane proteins that ligand binding during the functional cycle can alter the protein conformation (Ferguson et al., 2002; le Coutre et al., 2002; Wang, 1997) causing changes in molecular interactions. As the observed change in interactions is localized in the direct proximity of the ligand-binding site of NhaA, we applied force spectroscopy to probe the effect of $Na^+$ ions on this site. For this purpose we unfolded single NhaA molecules at pH 7.7 in absence of NaCl (Figure 1F). To eliminate possible effects of non-specific electrostatic interactions, the total ionic strength of the buffer solution was kept constant using KCl as substitute. The force experiments did not detect any change of the unfolding pathways upon absence of $Na^+$ ions except for the 225aa peak, which almost disappeared. Moreover, the distribution of unfolding forces (Figure 2D) exhibited two peaks at 60-70 pN and 90-100 pN. The peak at 90-100 pN suggested that a certain fraction of the molecules (30-35%) still possessed strong molecular interactions within helix V. The specificity of $Na^+$ ions strongly suggests that they play a major role in establishing the molecular interactions at the ligand binding site of helix V. Together with the observed pH-dependent formation of these interactions it may be suggested that the accessibility of the binding site to $Na^+$ ions is governed by pH. As shown recently, this ion binding capability can be altered upon small changes of the side-chain orientation (Wang, 1997), which is promoted by minute spatial rearrangements of NhaA helices. Thus we conclude that the antiporter is activated by intramolecular interactions, which are established only at neutral pH and simultaneously occurring ligand binding.

## Example 4:

*Molecular interactions establish full strength at high probability to activate ion channel*

To analyze formation and kinetics of molecular interactions that were established upon NhaA activation the frequency of peak appearance at aa 225 and rupture forces were measured at pH values ranging from 3.8 to 7.7. The data suggested that the interactions gradually increased when the pH was increased from 5 to 6 (Figure 3A). The probability of the peak appearance continuously increased with the pH as well (Figure 3B). It showed, however, a much lower slope beginning at pH 4 (~30%) and finally reaching ~95% at pH 7.5. Sigmoid distributions (Figures 3A, B; black curves) accurately fitted the data points assuming C1=39, C2=70 for the force and C1=29, C2=63 for the probability. Mid-points of both transitions were located at pH0= 5.4 (force) and 5.7 (probability). Clearly, both mid-points for establishing the molecular interactions were shifted to the acidic range, compared to pH 7.5 optimum reported for NhaA activity (Taglicht et al., 1991). One possible explanation could be that the observed formation of molecular interactions within transmembrane helix V relates to an early activation step of the protein, while previously reported structural changes (Rothman et al., 1997; Venturi et al., 2000) finalize the activation of the antiporter at pH range 7-8. The full activity of NhaA would then be reached at pH 7.0 at which the molecular interactions at the active site of the protein reached their full strength and occurred with a probability of > 90%. Thus, we conclude that establishing the full strength of molecular interactions builds an initial step towards activating a single NhaA. These observations are in agreement with those made on lactose permease (Abramson, 2003; Sun, 1998; Zhang, 2002), a paradigmatic secondary transporter (Abramson, 2004). Here it was shown that activation of this transporter is a multi-stage process. Thus, the observed molecular interactions may represent initial steps of conformational changes for NhaA, which have been previously observed at pH ranging between 7 and 8 (Rothman et al., 1997; Venturi et al., 2000). To activate all NhaA channels these molecular interactions have to be established in every NhaA molecule. This finding revealed by single molecule studies complements conventional experiments revealed from large protein assemblies. We conclude that apparently some proteins were activated by establishing their full strength of molecular interactions while other were not activated.

The unfolding spectra of membrane proteins significantly depend on the loading rate which reflects the non-equilibrium nature of the single-molecule experiments. As a result, the frequency of side peaks appearance can be modulated by pulling speed (Janovjak, 2004). Thus, we performed additional unfolding experiments on inactive NhaA (pH 3.8) at higher pulling speed of 1 um/s, which shifted the frequency of the 225aa peak to ~55%. In contrast, at pH 7.7 the frequency remained -95% (data available upon request). Remarkably, irrespective of the pulling speed the molecular interaction centered at aa 225 reached its maximum stability at pH - 6. Thus, we assume that changing the pulling speed shifts the siope of the molecular forces and of their probability to occur within the active site of NhaA. The conclusions remained unchanged, however, that the full strength of interactions is established at a pH range before NhaA becomes fully active.

**Example 5:**

In the following, the methods for carrying out examples 1 to 4 are described:

*NhaA preparation*

NhaA was overexpressed in *E.coli* BL21(DE3) with a $His_6$-tag fused to the C-terminus (Olami et al., 1997). Purification and 2D crystallization was performed at pH 4 (Williams et al., 1999), where the molecule is inactive. The tubular 2D crystals exhibited unit cell dimensions of 48Å x 181Å with a $p22_12_1$ symmetry. NhaA activity was determined measuring the active transport of $Na^+$ ions using the electrophysiological method of solid supported membranes (Seifert, 1993). The measured pH-profile of activation was identical to that obtained by transport measurements of $^{22}$Na (Taglicht et al., 1991).

*AFM*

The AFM used (Nanoscope IIIa) was equipped with a fluid cell and 200μm long $Si_3N_4$ AFM cantilevers (di-Veeco, Santa Barbara). Spring constants of cantilevers were determined (=0.06N/m) using the equipartition theorem (Butt H.J., 1995; Florin, 1995). 2D crystals of NhaA were immobilized on freshly cleaved mica in 150mM KCl, 10% glycerol, 25mM $Ka^+$-acetate, pH 4 for 20min. Experiments were performed in buffer solutions containing 150mM KCl and 50mM NaCl at pH 3.8 (20mM citric acid), 4.5 (20mM $K^+$-acetate), 5.0 (20mM citric acid), 5.5 (20mM MES), 6.3 (20mM HEPES), 7.1 (20mM HEPES), and 7.7 (20mM Tris). $Na^+$-free buffers contained less than 0.1 mM $Na^+$ as estimated by atomic absorption spectroscopy. All buffer solutions were made in fresh nano-pure water (18.2 MΩ•cm), using reagents from Sigma/Merck of p.a. purity grade. Upon buffer exchange the setup was equilibrated for 30min. After AFM imaging immobilized crystal patches (Kedrov, 2004) an unperturbed area was selected to unfold individual proteins. The AFM tip was then brought in contact with the protein applying a force of 0.5-1nN to attach its terminal end. After 1s the tip was withdrawn from the membrane at 120nm/s, while the cantilever deflection was detected. The value of the deflection at each time- point was used to calculate the force acting on the molecule via Hook's law.

*Data analysis*

Pulling NhaA (402aa) from either the N- or C-terminus yielded characteristic force-extension curves each exhibiting a length of ~100nm (Kedrov, 2004). In this study we focused on C-terminal unfolding events because of dramatic decrease in frequency of N-terminal unfolding events observed at higher pH. Force-extension curves recorded upon single-protein unfolding were manually superimposed. To obtain the unfolded polypeptide chain length each peak was fitted using the worm-like-chain (WLC) model (Bustamante C., 1994) as described (Kedrov, 2004).

Percent probability and average unfolding forces were calculated for each force peak. The standard error of the mean frequency value was derived from the binomial distribution. We analyzed 74 (pH 3.8), 43 (pH 4.5), 60 (pH 5.0), 70 (pH 5.5), 59 (pH 6.2), 68 (pH 7.1), 74 (pH 7.7) events at the pH indicated. Distributions of unfolding force and percent probabilities vs. pH were fitted using the sigmoid function described by

$$f(pH) = C_1 + \frac{C_2}{1 + \log^{-(pH - pH_0)}}$$

$C_1$, $C_2$ determine the limits of the function at low and high pH values, $pH_0$ the mid-point of the transition.

## References

Abramson J, Iwata S, Kaback HR (2004) Lactose permease as a paradigm for membrane transport proteins. Mol Membr Biol 21: 227-36 Abramson J, Smirnova I, Kasho V, Verner G, Kaback HR, Iwata S. (2003) Structure and mechanism of the lactose permease of Escherichia coli. Science 301: 610-5

Albrecht C et al. (2003) DNA: a programmable force sensor. Science 301: 367-70 Bustamante C, Siggia ED, Smith S (1994) Entropic elasticity of lambda-phage DNA. Science 265: 1599-600

Butt H.J (1995) Calculation of thermal noise in atomic force microscopy. Nanotechnology 6: 1-7

Cisneros D, Oesterhelt D, Muller DJ (2004) Probing origins of molecular interactions stabilizing the membrane proteins halorodopsin and bacteriorhodopsin. Structure in press

Ferguson AD, Chakraborty R, Smith BS, Esser L, Helm D, Deisenhofer J (2002) Structural basis of gating by the outer membrane transporter FecA. Science 295: 1715-9

Florin EL, Rief M, Lehmann H, Ludwig M, Dornmair C, Moy VT, Gaub HE (1995) Sensing specific molecular interactions with the atomic force microscopy. Biosens Bioelectr 10: 895-901 Galili L, Herz K, Dym O, Padan E (2004) Unraveling functional and structural interactions between transmembrane domains IV and XI of NhaA Na+/H+ antiporter of Escherichia coli. J Biol Chem 279: 23104-13 Galili L, Rothman A, Kozachkov L, Rimon A, Padan E (2002) Trans membrane domain IV is involved in ion transport activity and pH regulation of the NhaA-Na(+)/H(+) antiporter of Escherichia coli. Biochemistry 41: 609-17 Gerchman Y, Olami Y, Rimon A, Taglicht D, Schuldiner S, Padan E (1993) Histidine-226 is part of the pH sensor of NhaA, a Na+/H+ antiporter in Escherichia coli. Proc Natl Acad Sci USA 90: 1212-6 Gerchman Y, Rimon A, Padan E (1999) A pH-dependent conformational change of NhaA Na(+)/H(+) antiporter of Escherichia coli involves loop VIII-IX, plays a role in the pH response of the protein, and is maintained by the pure protein in dodecyl maltoside. J Biol Chem 274: 24617-24 Inoue H, Noumi T, Tsuchiya T, Kanazawa H (1995) Essential aspartic acid residues, Asp-133, Asp-163 and Asp-164, in the transmembrane helices of a Na+/H+ antiporter (NhaA) from Escherichia coli. FEBS Lett 363: 264-8

Janovjak H, Kessler M, Oesterhelt D, Gaub H, Muller DJ (2003) Unfolding pathways of native bacteriorhodopsin depend on temperature. Embo J 22: 5220-9 Janovjak H, Struckmeier J, Hubain M, Kedrov A, Kessler M, Muller DJ (2004). Probing the energy landscape of the membrane protein bacteriorhodopsin. Structure 12: 871-9

Karmazyn M, Gan XT, Humphreys RA, Yoshida H, Kusumoto K (1999) The myocardial Na(+)-H(+) exchange: structure, regulation, and its role in heart disease. Circul Res 85: 777-86

Kedrov A, Ziegler C., Janovjak H, Kuhlbrandt W, Muller DJ (2004) Controlled unfolding and refolding of a single sodium-proton antiporter using atomic force microscopy. J Mol Biol 340: 1143-52

le Coutre J, Turk E, Kaback HR, Wright EM (2002) Ligand-induced differences in secondary structure of the Vibrio parahaemolyticus Na+/galactose cotransporter. Biochemistry 41: 8082-6

Moller C, Fotiadis D, Suda K, Engel A, Kessler M, Muller DJ (2003) Determining molecular forces that stabilize human aquaporin-1. J Struct Biol 142: 369-78

Muller DJ, Kessler M, Oesterhelt F, Moller C, Oesterhelt D, Gaub H (2002) Stability of bacteriorhodopsin alpha-helices and loops analyzed by single-molecule force spectroscopy. Biophys J 83: 3578-88

Oesterhelt F, Oesterhelt D, Pfeiffer M, Engel A, Gaub HE, Muller DJ (2000) Unfolding pathways of individual bacteriorhodopsins. Science 288: 143-6

Olami Y, Rimon A, Gerchman Y, Rothman A, Padan E (1997) Histidine 225, a residue of the NhaA-Na+/H+ antiporter of Escherichia coli is exposed and faces the cell exterior. J Biol Chem 272: 1761-8

Padan E, Venturi M, Gerchman Y, Dover N (2001) Na(+)/H(+) antiporters. Biochim Biophys Acta 1505: 144-57

Ravna AW, Sylte I, Dahl SG (2001) Molecular model of the Escherichia coli Na1/H1 antiporter NhaA. Recept Channels 7: 319-28 Rimon A, Gerchman Y, Kariv Z, Padan E (1998) A point mutation (G338S) and its suppressor mutations affect both the pH response of the NhaA-Na+/H+ antiporter as well as the growth phenotype of Escherichia coli. J Biol Chem 273: 26470-6

Rothman A, Gerchman Y, Padan E, Schuldiner S (1997) Probing the conformation of NhaA, a Na+/H+ antiporter from Escherichia coli, with trypsin. Biochemistry 36: 14572-6

Rothman A, Padan E, Schuldiner S (1996) Topological analysis of NhaA, a Na+/H+ antiporter from Escherichia coli. J Biol Chem 271: 32288-92 Seifert K, Fendler K, Bamberg E (1993) Charge transport by ion translocating membrane proteins on solid supported membranes. Biophys J 64: 384-91

Sun J, Kemp CR, Kaback HR (1998) Ligand-induced changes in periplasmic loops in the lactose permease of Escherichia coli. Biochemistry 37: 8020-6 Taglicht D, Padan E, Schuldiner S (1991) Overproduction and purification of a functional Na+/H+ antiporter coded by nhaA (ant) from Escherichia coli. J Biol Chem 266: 11289-94

Tzubery T, Rimon A, Padan E (2004) Mutation E252C increases drastically the Km value for Na+ and causes an alkaline shift of the pH dependence of NhaA Na+/H+ antiporter of Escherichia coli. J Biol Chem 279: 3265-72

Venturi M, Rimon A, Gerchman Y, Hunte C, Padan E, Michel H (2000) The monoclonal antibody 1 F6 identifies a pH-dependent conformational change in the hydrophilic NH(2) terminus of NhaA Na(+)/H(+) antiporter of Escherichia coli. J Biol Chem 275: 4734-42 Wakabayashi S, Shigekawa M, Pouyssegur J (1997) Molecular physiology of vertebrate Na+/H+ exchangers. Physiol Rev 77: 51-74 Wang Q, Matsushita K, de Foresta B, le Maire M, Kaback HR (1997) Ligand-nduced movement of helix X in the lactose permease from E.coli: a fluorescence quenching study. Biochemistry 36: 14120-7

Williams KA (2000) Three-dimensional structure of the ion-coupled transport protein NhaA. Nature 403: 112-5

Williams KA, Geldmacher-Kaufer U, Padan E, Schuldiner S, Kuhlbrandt W (1999) Projection structure of NhaA, a secondary transporter from Escherichia coli, at 4.0 A resolution. Embo J 18: 3558-63 Zhang W, Guan L, Kaback HR (2002) Helices VII and X in the lactose permease of Escherichia coli: proximity and ligand-induced distance changes. J Mol Biol 315: 53-62

**Additional references:**

[14] Carrion-Vazquez M, Oberhauser AF, Fisher TE, Marszalek PE, Li H, Fernandez JM. Mechanical design of proteins studied by single-molecule force spectroscopy and protein engineering. Prog. Biophys. Mol. Biol. 2000;74:63-91.

[18] Cisneros DA, Oesterhelt D, Muller DJ. Probing origins of molecular interactions stabilizing the membrane proteins halorhodopsin and bacteriorhodopsin. Structure (Camb) 2005;13:235-42.

[22] Czajkowsky DM, Iwamoto H, Shao Z. Atomic force microscopy in structural biology: from the subcellular to the submolecular. J Electron Microsc (Tokyo) 2000;49:395-406.

[29] Drake B et al. Imaging crystals, polymers, and processes in water with the atomic force microscope. Science 1989;243:1586-1588.

[38] Fotiadis D, Scheuring S, Muller SA, Engel A, Muller DJ. Imaging and manipulation of biological structures with the AFM. Micron 2002;33:385-397.

[45] Frederix PL, Akiyama T, Staufer U, Gerber C, Fotiadis D, Muller DJ, Engel A. Atomic force bio-analytics. Curr Opin Chem Biol 2003;7:641-7.

[46] Frederix PT, Hoogenboom B, W., Fotiadis D, Müller DJ, Engel A. Atomic force microscopy of biological samples. MRS Bulletin 2004;29:449-455.

[57] Ikai A, Afrin R. Toward mechanical manipulations of cell membranes and membrane proteins using an atomic force microscope: an invited review. Cell Biochem Biophys 2003;39:257-77.

[59] Janovjak H, Kessler M, Gaub H, Oesterhelt D, Müller DJ. Unfolding pathways of native bacteriorhodopsin depend on temperature. EMBO J 2003;22: 5220-5229.

[60] Janovjak H, Struckmeier J, Hubain M, Kedrov A, Kessler M, Muller DJ. Probing the energy landscape of the membrane protein bacteriorhodopsin. Structure (Camb) 2004;12:871-9.

[64] Kedrov A, Ziegler C, Janovjak H, Kuhlbrandt W, Muller DJ. Controlled unfolding and refolding of a single sodium-proton antiporter using atomic force microscopy. J Mol Biol 2004;340:1143-1152.

[65] Kellermayer MS, Smith SB, Granzier HL, Bustamante C. Folding-unfolding transitions in single titin molecules characterized with laser tweezers. Science 1997;276:1112-1116.

[73] Leckband D. Measuring the forces that control protein interactions. Annu. Rev. Biophys. Biomol. Struct. 2000;29:1-26.

[79] Marszalek PE, Lu H, Li H, Carrion-Vazquez M, Oberhauser AF, Schulten K, Fernandez JM. Mechanical unfolding intermediates in titin modules. Nature 1999;402:100-103.

[85] Möller C, Fotiadis D, Suda K, Engel A, Kessler M, Müller DJ. Determining molecular forces that stabilize human aquaporin-1. J. Struct. Biol. 2003;142: 369-378.

[93] Müller DJ, Baumeister W, Engel A. Controlled unzipping of a bacterial surface layer with atomic force microscopy. Proc. Natl. Acad. Sci. U. S. A. 1999;96:13170-13174.

[98] Müller DJ, Kessler M, Oesterhelt F, Moeller C, Oesterhelt D, Gaub H. Stability of bacteriorhodopsin alpha-helices and loops analyzed by single-molecule force spectroscopy. Biophys. J. 2002;83: 3578-3588.

[103] Oesterhelt F, Oesterhelt D, Pfeiffer M, Engel A, Gaub HE, Müller DJ. Unfolding pathways of individual bacteriorhodopsins. Science 2000;288: 143-146.

[111] Rief M, Gautel M, Gaub HE. Unfolding forces of titin and fibronectin domains directly measured by AFM. Adv. Exp. Med. Biol. 2000;481:129-136.

[113] Rief M, Gautel M, Oesterhelt F, Fernandez JM, Gaub HE. Reversible unfolding of individual titin immunoglobulin domains by AFM. Science 1997;276: 1109-1112.

[115] Sapra KT, Besir H, Oesterhelt D, Muller DJ. Mechanisms stabilizing membrane proteins assembled into oligomers. EMBO J. 2005;submitted.

[149] Williams PM, Fowler SB, Best RB, Toca-Herrera JL, Scott KA, Steward A, Clarke J. Hidden complexity in the mechanical properties of titin. Nature 2003;422:446-449.

[150] Zhuang X, Rief M. Single-molecule folding. Curr Opin Struct Biol 2003;13:88-97.

**Claims**

1. A method for determining the state of activation of a peptide, polypeptide or protein, comprising the steps of:

   (a) immobilizing the peptide, polypeptide or protein on a carrier;
   (b) attaching the peptide, polypeptide or protein immobilized on the carrier to a force measuring device of a force spectroscope;
   (c) applying a pulling force to the peptide, polypeptide or protein and measuring the force required for stretching and/or unfolding of the peptide, polypeptide or protein attached to the carrier, wherein the measurements are carried out (i) prior to and (ii) after treatment with a known modulator of said peptide, polypeptide or protein;
   (d) comparing the force spectra of the peptide, polypeptide or protein prior to and after treatment with the known modulator; and
   (e) concluding from a difference of the force spectra of step (d) on the state of activation of the peptide, polypeptide or protein.

2. The method of claim 1, wherein the modulator is a known activator, inhibitor, co-factor, substrate or ligand of said peptide, polypeptide or protein.

3. A method for determining the state of activation of a peptide, polypeptide or protein, comprising the steps of:

   (a) immobilizing the peptide, polypeptide or protein on a carrier;
   (b) attaching the peptide, polypeptide or protein immobilized on the carrier to a force measuring device of a force spectroscope;
   (c) applying a pulling force to the peptide, polypeptide or protein and measuring the force required for stretching and/or unfolding of the peptide, polypeptide or protein attached to the carrier;
   (d) comparing the force spectra of the peptide, polypeptide or protein of (c) with a control representing an inactive or a known state of activation of the peptide, polypeptide or protein; and
   (e) concluding from a difference, if any, of the force spectra of step (d) on the state of activation of the peptide, polypeptide or protein.

4. A method for determining whether a compound is an activator of peptide, polypeptide or protein function, comprising the steps of:

   (a) immobilizing the peptide, polypeptide or protein on a carrier;
   (b) attaching the peptide, polypeptide or protein to a force measuring device of a force spectroscope;
   (c) applying a pulling force to the peptide, polypeptide or protein and measuring the force required for stretching and/or unfolding of the peptide, polypeptide or protein attached to the carrier, wherein separate measurements are carried out in the presence of a test and a reference buffer, wherein
   the test buffer comprises a compound suspected of being an activator of the function of said peptide, polypeptide or protein, and wherein
   the reference buffer comprises

   (i) a compound known to have no effect on the activity of said peptide, polypeptide or protein or
   (ii) a compound known to be an activator of said peptide, polypeptide or protein;

   (d) comparing the force spectra of the peptide, polypeptide or protein measured in the presence of the test and the reference buffer; and
   (e) concluding from a difference of the force spectra of step (d) whether the compound suspected of being an activator of said peptide, polypeptide or protein is an activator of the peptide, polypeptide or protein.

5. A method for determining whether a compound is an inhibitor of peptide, polypeptide or protein function, comprising the steps of:

   (a) immobilizing the peptide, polypeptide or protein on a carrier;
   (b) attaching the peptide, polypeptide or protein to a force measuring device of a force spectroscope;
   (c) applying a pulling force to the peptide, polypeptide or protein and measuring the force required for stretching and/or unfolding of the peptide, polypeptide or protein attached to the carrier, wherein separate measurements are carried out in the presence of a test and a reference buffer, wherein

the test buffer comprises a compound suspected of being an inhibitor of the function of said peptide, polypeptide or protein and wherein the reference buffer comprises

(i) a compound known to have no effect on the activity of said peptide, polypeptide or protein; or
(ii) a compound known to be an activator of said peptide, polypeptide or protein; or
(iii) a compound known to be an inhibitor of said peptide, polypeptide or protein;

(d) comparing the force spectra of the peptide, polypeptide or protein measured in the presence of the test and the reference buffer; and
(e) concluding from a difference of the force spectra of step (d) whether the compound suspected of being an inhibitor of said peptide, polypeptide or protein is an inhibitor of the peptide, polypeptide or protein.

6. The method of any one of claims 1 to 5, wherein the carrier is (a) a biological carrier selected from the group consisting of membrane, vesicle, cell, interface, biological structure, protein, nucleic acid molecule, fibril, fibre, and biological scaffold or (b) a non-biological carrier selected from the group consisting of solid state material, polymer, synthetic membrane and synthetic scaffold.

7. The method of any one of claims 1 to 6, wherein the peptide, polypeptide or protein is (a) arranged as a three-dimensional or two-dimensional crystal, (b) bound to a biological or non-biological surface, (c) attached to a membrane or peptide, (d) attached to any biological or synthetic molecule, or (e) incorporated into a membrane.

8. The method of any one of claims 1 to 7, wherein the peptide, polypeptide or protein is embedded in a lipid bilayer.

9. The method of any one of claims 1 to 8, wherein the measuring device is selected from (a) tip of a force spectroscope, (b) a magnetic tweezer, (c) an optical tweezer, (d) a protein or protein complex, (e) by a biological or synthetic molecule, (f) a surfaces of a force apparatus, (g) a membrane, and (h) the probe of a scanning probe microscope or a scanning probe spectroscope.

10. The method of any one of claims 1 to 9, wherein the peptide, polypeptide or protein is attached to the measuring device by non-covalent interactions.

11. The method of any one of claims 1 to 10, wherein the pulling force applied from the tip of the force spectroscope is in the range of 1 to 500 pN.

12. The method of any one of claims 1 to 11, wherein stretching and/or unfolding are measured by recording the unfolding and/or stretching resistance observed upon application of the pulling force from the tip of the force spectroscope to the peptide, polypeptide or protein.

13. The method of any one of claims 1 to 12, wherein said activator is an agonist.

14. The method of any one of claims 1 to 13, wherein said activator or inhibitor is selected from the group consisting of a protein specific ligand, a synthetic compound, a pharmaceutical compound, a biochemical or biological compound, lipid, peptide, polypeptide or protein, light, electrolyte, pH, voltage or current, being capable of inducing a change of the functional state or activity of said peptide, polypeptide or protein.

15. The method of any one of claims 2 to 14, wherein more than one compound suspected of being an activator or inhibitor of the peptide, polypeptide or protein function is tested.

16. The method of any one of claims 1 to 15, wherein the measurement is performed in the presence of a compound which is an agonist or antagonist of said activator or inhibitor.

17. The method of claim 15 or 16, wherein said compound is part of a compound library and wherein said method contains the additional step of screening said compound library for identifying a lead compound for drug development.

18. The method of claim 17, wherein said screening is high-throughput screening carried out by multiple cantilevers and/or by multiple force spectroscopes and/or by fast-speed force spectroscopy.

19. The method of any one of claims 1 to 18, wherein the peptide, polypeptide or protein is a protein.

20. The method of claim 19, wherein said protein is selected from the group consisting of (a) ligand-gated receptor, (b) G-protein coupled receptor, (c) ion channel, (d) water channel, (e) antiporter, (f) communication channel, (g) symporter, (h) porin, (i) ion gating channel, electrolyte gated channel and pore, ion pump, glutamate gated ion channel, ATP gated channel, (j) mechano-sensitive channel and (k) ATP synthase.

21. The method of any one of claims 1 to 20, wherein the pulling force is exerted by the measuring device.

22. The method of any one of claims 1 to 20, wherein the pulling force is exerted by the carrier.

23. The method of any one of claims 1 to 20, wherein the pulling force is exerted by the peptide, polypeptide or protein.

**Patentansprüche**

1. Verfahren zur Bestimmung des Zustands der Aktivierung eines Peptids, Polypeptids oder Proteins, das die Schritte umfasst:

a) Immobilisieren des Peptids, Polypeptids oder Proteins auf einem Träger;
b) Anbringen des auf dem Träger immobilisierten Peptids, Polypeptids oder Proteins an einer Kraftmesseinrichtung eines Kraftspektroskops;
c) Anwenden einer Zugkraft auf das Peptid, Polypeptid oder Protein und Messen der Kraft, die für das Strecken und/oder Entfalten des an dem Träger angebrachten Peptids, Polypeptids oder Proteins erforderlich ist; wobei die Messungen (i) vor und (ii) nach einer Behandlung mit einem bekannten Modulator des Peptids, Polypeptids oder Proteins durchgeführt werden;
d) Vergleichen der Kraftspektren des Peptids, Polypeptids oder Proteins vor und nach der Behandlung mit dem bekannten Modulator; und
e) Schließen auf den Zustand der Aktivierung des Peptids, Polypeptids oder Proteins aus einem Unterschied der Kraftspektren von Schritt (d).

2. Verfahren nach Anspruch 1, bei dem der Modulator ein bekannter bzw. bekanntes Aktivator, Inhibitor, Cofaktor, Substrat oder Ligand des Peptids, Polypeptids oder Proteins ist.

3. Verfahren zur Bestimmung des Zustands der Aktivierung eines Peptids, Polypeptids oder Proteins, das die Schritte umfasst:

a) Immobilisieren des Peptids, Polypeptids oder Proteins auf einem Träger;
b) Anbringen des auf dem Träger immobilisierten Peptids, Polypeptids oder Proteins an einer Kraftmesseinrichtung eines Kraftspektroskops;
c) Anwenden einer Zugkraft auf das Peptid, Polypeptid oder Protein und Messen der Kraft, die zum Strecken und/oder Entfalten des an dem Träger angebrachten Peptids, Polypeptids oder Proteins erforderlich ist;
d) Vergleichen der Kraftspektren des Peptids, Polypeptids oder Proteins von (c) mit einer Kontrolle, die einen inaktiven oder einen bekannten Zustand der Aktivierung des Peptids, Polypeptids oder Proteins darstellt; und
e) Schließen auf den Zustand der Aktivierung des Peptids, Polypeptids oder Proteins aus einem Unterschied, falls vorhanden, der Kraftspektren von Schritt (d).

4. Verfahren zur Bestimmung, ob eine Verbindung ein Aktivator einer Peptid-, Polypeptid- oder Protein-Funktion ist, das die Schritte umfasst:

a) Immobilisieren des Peptids, Polypeptids oder Proteins auf einem Träger;
b) Anbringen des Peptids, Polypeptids oder Proteins an einer Kraftmesseinrichtung eines Kraftspektroskops;
c) Anwenden einer Zugkraft auf das Peptid, Polypeptid oder Protein und Messen der Kraft, die zum Strecken und/oder Entfalten des an dem Träger angebrachten Peptids, Polypeptids oder Proteins erforderlich ist, wobei getrennte Messungen in Anwesenheit eines Test- und eines Bezugspuffers durchgeführt werden, wobei der Testpuffer eine Verbindung umfasst, von der angenommen wird, dass sie ein Aktivator der Funktion des Peptids, Polypeptids oder Proteins ist, und wobei der Bezugspuffer

(i) eine Verbindung, von der bekannt ist, dass sie keine Auswirkung auf die Aktivität des Peptids, Polypeptids oder Proteins hat, oder
(ii) eine Verbindung, von der bekannt ist, dass sie ein Aktivator des Peptids, Polypeptids oder Proteins ist,

umfasst;
d) Vergleichen der Kraftspektren des Peptids, Polypeptids oder Proteins, die in Anwesenheit des Test- und des Bezugspuffers gemessen wurden; und
e) Schließen aus einem Unterschied der Kraftspektren von (d), ob die Verbindung, von der angenommen wird, dass sie ein Aktivator des Peptids, Polypeptids oder Proteins ist, ein Aktivator des Peptids, Polypeptids oder Proteins ist.

5. Verfahren zur Bestimmung, ob eine Verbindung ein Inhibitor einer Peptid-, Polypeptid- oder Protein-Funktion ist, das die Schritte umfasst:

a) Immobilisieren des Peptids, Polypeptids oder Proteins auf einem Träger;
b) Anbringen des Peptids, Polypeptids oder Proteins an einer Kraftmesseinrichtung eines Kraftspektroskops;
c) Anwenden einer Zugkraft auf das Peptid, Polypeptid oder Protein und Messen der Kraft, die erforderlich ist, um das an dem Träger angebrachte Peptid, Polypeptid oder Protein zu strecken und/oder zu entfalten, wobei getrennte Messungen in Anwesenheit eines Test- und eines Bezugspuffers durchgeführt werden, wobei der Testpuffer eine Verbindung umfasst, von der angenommen wird, dass sie ein Inhibitor der Funktion des Peptids, Polypeptids oder Proteins ist, und wobei
der Bezugspuffer

(i) eine Verbindung, von der bekannt ist, dass sie keine Auswirkung auf die Aktivität des Peptids, Polypeptids oder Proteins aufweist, oder
(ii) eine Verbindung, von der bekannt ist, dass sie ein Aktivator des Peptids, Polypeptids oder Proteins ist, oder
(iii) eine Verbindung, von der bekannt ist, dass sie ein Inhibitor des Peptids, Polypeptids oder Proteins ist,

umfasst;
d) Vergleichen der Kraftspektren des Peptids, Polypeptids oder Proteins, die in Anwesenheit des Test- und des Bezugspuffers gemessen wurden; und
e) Schließen aus einem Unterschied der Kraftspektren von (d), ob die Verbindung, von der angenommen wird, dass sie ein Inhibitor des Peptids, Polypeptids oder Proteins ist, ein Inhibitor des Peptids, Polypeptids oder Proteins ist.

6. Verfahren nach irgendeinem der Ansprüche 1 bis 5, bei dem der Träger (a) ein biologischer Träger ist, der ausgewählt ist aus der Gruppe bestehend aus Membran, Vesikel, Zelle, Grenzfläche, biologischer Struktur, Protein, Nukleinsäure-Molekül, Fibrille, Faser und biologischem Gerüst, oder (b) ein nichtbiologischer Träger ist, der ausgewählt ist aus der Gruppe bestehend aus festem Material, Polymer, synthetischer Membran und synthetischem Gerüst.

7. Verfahren nach irgendeinem der Ansprüche 1 bis 6, bei dem das Peptid, Polypeptid oder Protein (a) als zweidimensionaler oder dreidimensionaler Kristall angeordnet ist, (b) an eine biologische oder nichtbiologische Oberfläche gebunden ist, (c) an einer Membran oder einem Peptid angebracht ist, (d) an irgendeinem biologischen oder synthetischen Molekül angebracht ist oder (e) einer Membran einverleibt ist.

8. Verfahren nach irgendeinem der Ansprüche 1 bis 7, bei dem das Peptid, Polypeptid oder Protein in eine Lipid-Doppelschicht eingebettet ist.

9. Verfahren nach irgendeinem der Ansprüche 1 bis 8, bei dem die Messeinrichtung ausgewählt ist aus (a) der Spitze eines Kraftspektroskops, (b) einer magnetischen Pinzette, (c) einer optischen Pinzette, (d) einem Protein oder Protein-Komplex, (e) einem biologischen oder synthetischen Molekül, (f) einer Oberfläche einer Kraftvorrichtung, (g) einer Membran und (h) der Sonde eines Rastersondenmikroskops oder eines Rastersondenspektroskops.

10. Verfahren nach irgendeinem der Ansprüche 1 bis 9, bei dem das Peptid, Polypeptid oder Protein durch nicht-kovalente Wechselwirkungen an der Messeinrichtung angebracht ist.

11. Verfahren nach irgendeinem der Ansprüche 1 bis 10, bei dem die Zugkraft, die von der Spitze des Kraftspektroskops angewendet wird, im Bereich von 1 bis 500 pN liegt.

12. Verfahren nach irgendeinem der Ansprüche 1 bis 11, bei dem das Strecken und/oder Entfalten durch Aufzeichnung des Entfaltungs- und/oder Streckwiderstands, der bei Anwendung der Zugkraft von der Spitze des Kraftspektroskops auf das Peptid, Polypeptid oder Protein beobachtet wird, gemessen werden.

13. Verfahren nach irgendeinem der Ansprüche 1 bis 12, bei dem der Aktivator ein Agonist ist.

14. Verfahren nach irgendeinem der Ansprüche 1 bis 13, bei dem der Aktivator oder Inhibitor ausgewählt ist aus der Gruppe bestehend aus einem Proteinspezifischen Liganden, einer synthetischen Verbindung, einer pharmazeutischen Verbindung, einer biochemischen oder biologischen Verbindung, einem Lipid, einem Peptid, einem Polypeptid oder Protein, Licht, einem Elektrolyt, dem pH, Spannung oder Strom, welche(r) bzw. welches in der Lage ist, eine Änderung des funktionellen Zustands oder der Aktivität des Peptids, Polypeptids oder Proteins zu induzieren.

15. Verfahren nach irgendeinem der Ansprüche 2 bis 14, bei dem mehr als eine Verbindung, von der angenommen wird, dass sie ein Aktivator oder Inhibitor der Peptid-, Polypeptid- oder Proteinfunktion ist, getestet wird.

16. Verfahren nach irgendeinem der Ansprüche 1 bis 15, bei dem die Messung in Anwesenheit einer Verbindung durchgeführt wird, die ein Agonist oder Antagonist des Aktivators oder Inhibitors ist.

**17.** Verfahren nach Anspruch 15 oder 16, bei dem die Verbindung ein Teil einer Verbindungsbibliothek ist, und bei dem das Verfahren den zusätzlichen Schritt der Durchmusterung der Verbindungsbibliothek zur Identifizierung einer Lead-Verbindung für eine Arzneistoffentwicklung enthält.

**18.** Verfahren nach Anspruch 17, bei dem die Durchmusterung eine Hochdurchsatz-Durchmusterung ist, die von mehreren Auslegern und/oder mehreren Kraftspektroskopen und/oder durch Hochgeschwindigkeits-Kraftspektroskopie durchgeführt wird.

**19.** Verfahren nach irgendeinem der Ansprüche 1 bis 18, bei dem das Peptid, Polypeptid oder Protein ein Protein ist.

**20.** Verfahren nach Anspruch 19, bei dem das Protein ausgewählt ist aus der Gruppe bestehend aus (a) Liganden-gesteuertem Rezeptor, (b) G-Proteingekoppeltem Rezeptor, (c) Ionenkanal, (d) Wasserkanal, (e) Antiporter, (f) Kommunikationskanal, (g) Symporter, (h) Porin, (i) Ionen-gesteuertem Kanal, Elektrolyt-gesteuertem Kanal und Pore, Ionenpumpe, Glutamat-gesteuertem Ionenkanal, ATP-gesteuertem Kanal, (j) mechano-sensitivem Kanal und (k) ATP-Synthase.

**21.** Verfahren nach irgendeinem der Ansprüche 1 bis 20, bei dem die Zugkraft von der Messvorrichtung ausgeübt wird.

**22.** Verfahren nach irgendeinem der Ansprüche 1 bis 20, bei dem die Zugkraft von dem Träger ausgeübt wird.

**23.** Verfahren nach irgendeinem der Ansprüche 1 bis 20, bei dem die Zugkraft von dem Peptid, Polypeptid oder Protein ausgeübt wird.

**Revendications**

**1.** Méthode pour déterminer de l'état d'activation d'un peptide, d'un polypeptide ou d'une protéine comprenant les étapes :

(a) immobiliser le peptide, le polypeptide ou la protéine sur un support ;
(b) attacher le peptide, le polypeptide ou la protéine immobilisé(e) sur le support à un dispositif de mesure de la force d'un spectroscope de force ;
(c) appliquer une force de traction sur le peptide, le polypeptide ou la protéine et mesurer la force requise pour étirer et/ou déplier le peptide, le polypeptide ou la protéine attaché(e) au support, les mesures étant effectuées (i) avant et (ii) après traitement avec un modulateur connu dudit peptide, dudit polypeptide ou de ladite protéine ;
(d) comparer les spectres de force du peptide, du polypeptide ou de la protéine avant et après traitement avec le modulateur connu ; et
(e) en conclure, d'après une différence des spectres de force de l'étape (d), l'état d'activation du peptide, du polypeptide ou de la protéine.

**2.** Méthode selon la revendication 1, dans laquelle le modulateur est un activateur, un inhibiteur, un cofacteur, un substrat ou un ligand connu dudit peptide, dudit polypeptide ou de ladite protéine.

**3.** Méthode pour déterminer de l'état d'activation d'un peptide, d'un polypeptide ou d'une protéine comprenant les étapes :

(a) immobiliser le peptide, le polypeptide ou la protéine sur un support ;
(b) attacher le peptide, le polypeptide ou la protéine immobilisé(e) sur le support à un dispositif de mesure de la force d'un spectroscope de force ;
(c) appliquer une force de traction sur le peptide, le polypeptide ou la protéine et mesurer la force requise pour étirer et/ou déplier le peptide, le polypeptide ou la protéine attaché(e) au support ;
(d) comparer les spectres de force du peptide, du polypeptide ou de la protéine de (c) avec un contrôle représentant un état d'activation inactif ou connu du peptide, du polypeptide ou de la protéine ; et
(e) en conclure, d'après une différence le cas échéant des spectres de force de l'étape (d), l'état d'activation du peptide, du polypeptide ou de la protéine.

**4.** Méthode pour déterminer si un composé est un activateur de la fonction du peptide, du polypeptide ou de la protéine comprenant les étapes :

(a) immobiliser le peptide, le polypeptide ou la protéine sur un support ;
(b) attacher le peptide, le polypeptide ou la protéine à un dispositif de mesure de la force d'un spectroscope de force ;
(c) appliquer une force de traction sur le peptide, le polypeptide ou la protéine et mesurer la force requise pour étirer et/ou déplier le peptide, le polypeptide ou la protéine attaché(e) au support, des mesures distinctes étant effectuées en présence d'un tampon d'essai et d'un tampon de référence,
le tampon d'essai comprenant un composé suspecté d'être un activateur de la fonction dudit peptide, dudit polypeptide ou de ladite protéine, et

le tampon de référence comprenant

(i) un composé connu pour n'avoir aucun effet sur l'activité dudit peptide, dudit polypeptide ou de ladite protéine ou

(ii) un composé connu pour être un activateur dudit peptide, dudit polypeptide ou de ladite protéine ;

(d) comparer les spectres de force du peptide, du polypeptide ou de la protéine mesurés en présence des tampons d'essai et de référence ; et

(e) en conclure, d'après une différence des spectres de force de l'étape (d), si le composé suspecté d'être un activateur dudit peptide, dudit polypeptide ou de ladite protéine est un activateur du peptide, du polypeptide ou de la protéine.

5. Méthode pour déterminer si un composé est un inhibiteur de la fonction du peptide, du polypeptide ou de la protéine comprenant les étapes :

(a) immobiliser le peptide, le polypeptide ou la protéine sur un support ;

(b) attacher le peptide, le polypeptide ou la protéine à un dispositif de mesure de la force d'un spectroscope de force ;

(c) appliquer une force de traction sur le peptide, le polypeptide ou la protéine et mesurer la force requise pour étirer et/ou déplier le peptide, le polypeptide ou la protéine attaché(e) au support, des mesures distinctes étant effectuées en présence d'un tampon d'essai et d'un tampon de référence,

le tampon d'essai comprenant un composé suspecté d'être un inhibiteur de la fonction dudit peptide, dudit polypeptide ou de ladite protéine, et

le tampon de référence comprenant

(i) un composé connu pour n'avoir aucun effet sur l'activité dudit peptide, dudit polypeptide ou de ladite protéine ; ou

(ii) un composé connu pour être un activateur dudit peptide, dudit polypeptide ou de ladite protéine ; ou

(iii) un composé connu pour être un inhibiteur dudit peptide, dudit polypeptide ou de ladite protéine ;

(d) comparer les spectres de force du peptide, du polypeptide ou de la protéine mesurés en présence du tampon d'essai et du tampon de référence ; et

(e) en conclure, d'après une différence des spectres de force de l'étape (d), si le composé suspecté d'être un inhibiteur dudit peptide, dudit polypeptide ou de ladite protéine est un inhibiteur du peptide, du polypeptide ou de la protéine.

6. Méthode selon l'une quelconque des revendications 1 à 5, dans laquelle le support est (a) un support biologique choisi dans le groupe constitué d'une membrane, d'une vésicule, d'une cellule, d'une interface, d'une structure biologique, d'une protéine, d'une molécule d'acide nucléique, d'une fibrille, d'une fibre et d'un treillis biologique ou (b) un support non biologique choisi dans le groupe constitué d'une matière à l'état solide, d'un polymère, d'une membrane synthétique et d'un treillis synthétique.

7. Méthode selon l'une quelconque des revendications 1 à 6, dans laquelle le peptide, le polypeptide ou la protéine est (a) agencé(e) sous forme de cristal tridimensionnel ou bi-dimensionnel, (b) fixé(e) à une surface biologique ou non biologique, (c) attaché(e) à une membrane ou à un peptide, (d) attaché(e) à n'importe quelle molécule biologique ou synthétique, ou (e) incorporé(e) dans une membrane.

8. Méthode selon l'une quelconque des revendications 1 à 7, dans laquelle le peptide, le polypeptide ou la protéine est intégré(e) dans une bicouche lipidique.

9. Méthode selon l'une quelconque des revendications 1 à 8, dans laquelle le dispositif de mesure est choisi parmi (a) la pointe d'un spectroscope de force, (b) une pince magnétique, (c) une pince optique, (d) une protéine ou un complexe protéique, (e) une molécule biologique ou synthétique, (f) la surface d'un dispositif de force, (g) une membrane et (h) la sonde d'un microscope en champ proche ou d'un spectroscope en champ proche.

10. Méthode selon l'une quelconque des revendications 1 à 9, dans laquelle le peptide, le polypeptide ou la protéine est attaché(e) au dispositif de mesure par des interactions non covalentes.

11. Méthode selon l'une quelconque des revendications 1 à 10, dans laquelle la force de traction appliquée depuis la pointe du spectroscope de force se situe dans la gamme de 1 à 500 pN.

12. Méthode selon l'une quelconque des revendications 1 à 11, dans laquelle l'étirement et/ou le dépliage sont mesurés en enregistrant la résistance au dépliage et/ou à l'étirement observée à l'application de la force de traction à partir de la pointe du spectroscope de force au peptide, au polypeptide ou à la protéine.

**13.** Méthode selon l'une quelconque des revendications 1 à 12, dans laquelle ledit activateur est un agoniste.

**14.** Méthode selon l'une quelconque des revendications 1 à 13, dans laquelle ledit activateur ou ledit inhibiteur est choisi dans le groupe constitué d'un ligand spécifique de protéines, d'un composé synthétique, d'un composé pharmaceutique, d'un composé biochimique ou biologique, lipide, peptide, polypeptide ou protéine, d'une lumière, d'un électrolyte, du pH, d'une tension ou d'un courant capable d'induire un changement dans l'état ou l'activité fonctionnel(le) dudit peptide, dudit polypeptide ou de ladite protéine.

**15.** Méthode selon l'une quelconque des revendications 2 à 14, dans laquelle plus d'un composé suspecté d'être un activateur ou un inhibiteur de la fonction du peptide, du polypeptide ou de la protéine est testé.

**16.** Méthode selon l'une quelconque des revendications 1 à 15, dans laquelle la mesure est effectuée en présence d'un composé qui est un agoniste ou un antagoniste dudit activateur ou inhibiteur.

**17.** Méthode selon la revendication 15 ou 16, dans laquelle ledit composé fait partie d'une banque de composés et dans laquelle ladite méthode contient l'étape additionnelle de criblage de ladite banque de composés pour identifier un composé principal pour le développement de médicaments.

**18.** Méthode selon la revendication 17, dans laquelle ledit criblage est un criblage à haut débit effectué par plusieurs cantilevers et/ou par plusieurs spectroscopes de force et/ou par spectroscopie de force à grande vitesse.

**19.** Méthode selon l'une quelconque des revendications 1 à 18, dans laquelle le peptide, le polypeptide ou la protéine est une protéine.

**20.** Méthode selon la revendication 19, dans laquelle ladite protéine est choisie dans le groupe constitué (a) d'un récepteur ligand-dépendant, (b) d'un récepteur couplé à une protéine G, (c) d'un canal ionique, (d) d'un canal hydrique, (e) d'un antiport, (f) d'un canal de communication, (g) d'un symport, (h) d'une porine, (i) d'un canal ion-dépendant, d'un canal électrolyte-dépendant, et d'un pore, d'une pompe ionique, d'un canal ionique glutamate-dépendant, d'un canal ATP-dépendant, (j) d'un canal mécanosensible et (k) de l'ATP-synthase.

**21.** Méthode selon l'une quelconque des revendications 1 à 20, dans laquelle la force de traction est exercée par le dispositif de mesure.

**22.** Méthode selon l'une quelconque des revendications 1 à 20, dans laquelle la force de traction est exercée par le support.

**23.** Méthode selon l'une quelconque des revendications 1 à 20, dans laquelle la force de traction est exercée par le peptide, le polypeptide ou la protéine.

Figure 1

**Figure 2**

**Figure 3**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MÜLLER, D. et al.** *Biophysical Journal,* 2002, vol. 83, 3578-3588
- **FREDERIX PLTM et al.** *Curr. Op. Chem. Biol.,* 2003, vol. 7, 641-647
- **ABRAMSON J ; IWATA S ; KABACK HR.** Lactose permease as a paradigm for membrane transport proteins. *Mol Membr Biol,* 2004, vol. 21, 227-36
- **ABRAMSON J ; SMIRNOVA I ; KASHO V ; VERNER G ; KABACK HR ; IWATA S.** Structure and mechanism of the lactose permease of Escherichia coli. *Science,* 2003, vol. 301, 610-5
- **ALBRECHT C et al.** DNA: a programmable force sensor. *Science,* 2003, vol. 301, 367-70
- **BUSTAMANTE C ; SIGGIA ED ; SMITH S.** Entropic elasticity of lambda-phage DNA. *Science,* 1994, vol. 265, 1599-600
- **BUTT H.J.** Calculation of thermal noise in atomic force microscopy. *Nanotechnology,* 1995, vol. 6, 1-7
- **CISNEROS D ; OESTERHELT D ; MULLER DJ.** Probing origins of molecular interactions stabilizing the membrane proteins halorodopsin and bacteriorhodopsin. *Structure,* 2004
- **FERGUSON AD ; CHAKRABORTY R ; SMITH BS ; ESSER L ; HELM D ; DEISENHOFER J.** Structural basis of gating by the outer membrane transporter FecA. *Science,* 2002, vol. 295, 1715-9
- **FLORIN EL ; RIEF M ; LEHMANN H ; LUDWIG M ; DORNMAIR C ; MOY VT ; GAUB HE.** Sensing specific molecular interactions with the atomic force microscopy. *Biosens Bioelectr,* 1995, vol. 10, 895-901
- **GALILI L ; HERZ K ; DYM O ; PADAN E.** Unraveling functional and structural interactions between transmembrane domains IV and XI of NhaA Na+/H+ antiporter of Escherichia coli. *J Biol Chem,* 2004, vol. 279, 23104-13
- **GALILI L ; ROTHMAN A ; KOZACHKOV L ; RIMON A ; PADAN E.** Trans membrane domain IV is involved in ion transport activity and pH regulation of the NhaA-Na(+)/H(+) antiporter of Escherichia coli. *Biochemistry,* 2002, vol. 41, 609-17
- **GERCHMAN Y ; OLAMI Y ; RIMON A ; TAGLICHT D ; SCHULDINER S ; PADAN E.** Histidine-226 is part of the pH sensor of NhaA, a Na+/H+ antiporter in Escherichia coli. *Proc Natl Acad Sci USA,* 1993, vol. 90, 1212-6
- **GERCHMAN Y ; RIMON A ; PADAN E.** A pH-dependent conformational change of NhaA Na(+)/H(+) antiporter of Escherichia coli involves loop VIII-IX, plays a role in the pH response of the protein, and is maintained by the pure protein in dodecyl maltoside. *J Biol Chem,* 1999, vol. 274, 24617-24
- **INOUE H ; NOUMI T ; TSUCHIYA T ; KANAZAWA H.** Essential aspartic acid residues, Asp-133, Asp-163 and Asp-164, in the transmembrane helices of a Na+/H+ antiporter (NhaA) from Escherichia coli. *FEBS Lett,* 1995, vol. 363, 264-8
- **JANOVJAK H ; KESSLER M ; OESTERHELT D ; GAUB H ; MULLER DJ.** Unfolding pathways of native bacteriorhodopsin depend on temperature. *Embo J,* 2003, vol. 22, 5220-9
- **JANOVJAK H ; STRUCKMEIER J ; HUBAIN M ; KEDROV A ; KESSLER M ; MULLER DJ.** Probing the energy landscape of the membrane protein bacteriorhodopsin. *Structure,* 2004, vol. 12, 871-9
- **KARMAZYN M ; GAN XT ; HUMPHREYS RA ; YOSHIDA H ; KUSUMOTO K.** The myocardial Na(+)-H(+) exchange: structure, regulation, and its role in heart disease. *Circul Res,* 1999, vol. 85, 777-86
- **KEDROV A ; ZIEGLER C. ; JANOVJAK H ; KUHLBRANDT W ; MULLER DJ.** Controlled unfolding and refolding of a single sodium-proton antiporter using atomic force microscopy. *J Mol Biol,* 2004, vol. 340, 1143-52
- **LE COUTRE J ; TURK E ; KABACK HR ; WRIGHT EM.** Ligand-induced differences in secondary structure of the Vibrio parahaemolyticus Na+/galactose cotransporter. *Biochemistry,* 2002, vol. 41, 8082-6
- **MOLLER C ; FOTIADIS D ; SUDA K ; ENGEL A ; KESSLER M ; MULLER DJ.** Determining molecular forces that stabilize human aquaporin-1. *J Struct Biol,* 2003, vol. 142, 369-78
- **MULLER DJ ; KESSLER M ; OESTERHELT F ; MOLLER C ; OESTERHELT D ; GAUB H.** Stability of bacteriorhodopsin alpha-helices and loops analyzed by single-molecule force spectroscopy. *Biophys J,* 2002, vol. 83, 3578-88
- **OESTERHELT F ; OESTERHELT D ; PFEIFFER M ; ENGEL A ; GAUB HE ; MULLER DJ.** Unfolding pathways of individual bacteriorhodopsins. *Science,* 2000, vol. 288, 143-6

- **OLAMI Y ; RIMON A ; GERCHMAN Y ; ROTHMAN A ; PADAN E.** Histidine 225, a residue of the NhaA-Na+/H+ antiporter of Escherichia coli is exposed and faces the cell exterior. *J Biol Chem,* 1997, vol. 272, 1761-8
- **PADAN E ; VENTURI M ; GERCHMAN Y ; DOVER N.** Na(+)/H(+) antiporters. *Biochim Biophys Acta,* 2001, vol. 1505, 144-57
- **RAVNA AW ; SYLTE I ; DAHL SG.** Molecular model of the Escherichia coli Na1/H1 antiporter NhaA. *Recept Channels,* 2001, vol. 7, 319-28
- **RIMON A ; GERCHMAN Y ; KARIV Z ; PADAN E.** A point mutation (G338S) and its suppressor mutations affect both the pH response of the NhaA-Na+/H+ antiporter as well as the growth phenotype of Escherichia coli. *J Biol Chem,* 1998, vol. 273, 26470-6
- **ROTHMAN A ; GERCHMAN Y ; PADAN E ; SCHULDINER S.** Probing the conformation of NhaA, a Na+/H+ antiporter from Escherichia coli, with trypsin. *Biochemistry,* 1997, vol. 36, 14572-6
- **ROTHMAN A ; PADAN E ; SCHULDINER S.** Topological analysis of NhaA, a Na+/H+ antiporter from Escherichia coli. *J Biol Chem,* 1996, vol. 271, 32288-92
- **SEIFERT K ; FENDLER K ; BAMBERG E.** Charge transport by ion translocating membrane proteins on solid supported membranes. *Biophys J,* 1993, vol. 64, 384-91
- **SUN J ; KEMP CR ; KABACK HR.** Ligand-induced changes in periplasmic loops in the lactose permease of Escherichia coli. *Biochemistry,* 1998, vol. 37, 8020-6
- **TAGLICHT D ; PADAN E ; SCHULDINER S.** Overproduction and purification of a functional Na+/H+ antiporter coded by nhaA (ant) from Escherichia coli. *J Biol Chem,* 1991, vol. 266, 11289-94
- **TZUBERY T ; RIMON A ; PADAN E.** Mutation E252C increases drastically the Km value for Na+ and causes an alkaline shift of the pH dependence of NhaA Na+/H+ antiporter of Escherichia coli. *J Biol Chem,* 2004, vol. 279, 3265-72
- **VENTURI M ; RIMON A ; GERCHMAN Y ; HUNTE C ; PADAN E ; MICHEL H.** The monoclonal antibody 1 F6 identifies a pH-dependent conformational change in the hydrophilic NH(2) terminus of NhaA Na(+)/H(+) antiporter of Escherichia coli. *J Biol Chem,* 2000, vol. 275, 4734-42
- **WAKABAYASHI S ; SHIGEKAWA M ; POUYSSEGUR J.** Molecular physiology of vertebrate Na+/H+ exchangers. *Physiol Rev,* 1997, vol. 77, 51-74
- **WANG Q ; MATSUSHITA K ; DE FORESTA B ; LE MAIRE M ; KABACK HR.** Ligand-nduced movement of helix X in the lactose permease from E.coli: a fluorescence quenching study. *Biochemistry,* 1997, vol. 36, 14120-7
- **WILLIAMS KA.** Three-dimensional structure of the ion-coupled transport protein NhaA. *Nature,* 2000, vol. 403, 112-5
- **WILLIAMS KA ; GELDMACHER-KAUFER U ; PADAN E ; SCHULDINER S ; KUHLBRANDT W.** Projection structure of NhaA, a secondary transporter from Escherichia coli, at 4.0 A resolution. *Embo J,* 1999, vol. 18, 3558-63
- **ZHANG W ; GUAN L ; KABACK HR.** Helices VII and X in the lactose permease of Escherichia coli: proximity and ligand-induced distance changes. *J Mol Biol,* 2002, vol. 315, 53-62
- **CARRION-VAZQUEZ M ; OBERHAUSER AF ; FISHER TE ; MARSZALEK PE ; LI H ; FERNANDEZ JM.** Mechanical design of proteins studied by single-molecule force spectroscopy and protein engineering. *Prog. Biophys. Mol. Biol.,* 2000, vol. 74, 63-91
- **CISNEROS DA ; OESTERHELT D ; MULLER DJ.** Probing origins of molecular interactions stabilizing the membrane proteins halorhodopsin and bacteriorhodopsin. *Structure (Camb,* 2005, vol. 13, 235-42
- **CZAJKOWSKY DM ; IWAMOTO H ; SHAO Z.** Atomic force microscopy in structural biology: from the subcellular to the submolecular. *J Electron Microsc (Tokyo,* 2000, vol. 49, 395-406
- **DRAKE B et al.** Imaging crystals, polymers, and processes in water with the atomic force microscope. *Science,* 1989, vol. 243, 1586-1588
- **FOTIADIS D ; SCHEURING S ; MULLER SA ; ENGEL A ; MULLER DJ.** Imaging and manipulation of biological structures with the AFM. *Micron,* 2002, vol. 33, 385-397
- **FREDERIX PL ; AKIYAMA T ; STAUFER U ; GERBER C ; FOTIADIS D ; MULLER DJ ; ENGEL A.** Atomic force bio-analytics. *Curr Opin Chem Biol,* 2003, vol. 7, 641-7
- **FREDERIX PT ; HOOGENBOOM B, W. ; FOTIADIS D ; MÜLLER DJ ; ENGEL A.** Atomic force microscopy of biological samples. *MRS Bulletin,* 2004, vol. 29, 449-455
- **IKAI A ; AFRIN R.** Toward mechanical manipulations of cell membranes and membrane proteins using an atomic force microscope: an invited review. *Cell Biochem Biophys,* 2003, vol. 39, 257-77
- **JANOVJAK H ; KESSLER M ; GAUB H ; OESTERHELT D ; MÜLLER DJ.** Unfolding pathways of native bacteriorhodopsin depend on temperature. *EMBO J,* 2003, vol. 22, 5220-5229
- **JANOVJAK H ; STRUCKMEIER J ; HUBAIN M ; KEDROV A ; KESSLER M ; MULLER DJ.** Probing the energy landscape of the membrane protein bacteriorhodopsin. *Structure (Camb,* 2004, vol. 12, 871-9

- **KEDROV A ; ZIEGLER C ; JANOVJAK H ; KUHL-BRANDT W ; MULLER DJ.** Controlled unfolding and refolding of a single sodium-proton antiporter using atomic force microscopy. *J Mol Biol,* 2004, vol. 340, 1143-1152
- **KELLERMAYER MS ; SMITH SB ; GRANZIER HL ; BUSTAMANTE C.** Folding-unfolding transitions in single titin molecules characterized with laser tweezers. *Science,* 1997, vol. 276, 1112-1116
- **LECKBAND D.** Measuring the forces that control protein interactions. *Annu. Rev. Biophys. Biomol. Struct.,* 2000, vol. 29, 1-26
- **MARSZALEK PE ; LU H ; LI H ; CARRION-VAZQUEZ M ; OBERHAUSER AF ; SCHULTEN K ; FERNANDEZ JM.** Mechanical unfolding intermediates in titin modules. *Nature,* 1999, vol. 402, 100-103
- **MÖLLER C ; FOTIADIS D ; SUDA K ; ENGEL A ; KESSLER M ; MÜLLER DJ.** Determining molecular forces that stabilize human aquaporin-1. *J. Struct. Biol.,* 2003, vol. 142, 369-378
- **MÜLLER DJ ; BAUMEISTER W ; ENGEL A.** Controlled unzipping of a bacterial surface layer with atomic force microscopy. *Proc. Natl. Acad. Sci. U. S. A.,* 1999, vol. 96, 13170-13174
- **MÜLLER DJ ; KESSLER M ; OESTERHELT F ; MOELLER C ; OESTERHELT D ; GAUB H.** Stability of bacteriorhodopsin alpha-helices and loops analyzed by single-molecule force spectroscopy. *Biophys. J.,* 2002, vol. 83, 3578-3588
- **OESTERHELT F ; OESTERHELT D ; PFEIFFER M ; ENGEL A ; GAUB HE ; MÜLLER DJ.** Unfolding pathways of individual bacteriorhodopsins. *Science,* 2000, vol. 288, 143-146
- **RIEF M ; GAUTEL M ; GAUB HE.** Unfolding forces of titin and fibronectin domains directly measured by AFM. *Adv. Exp. Med. Biol.,* 2000, vol. 481, 129-136
- **RIEF M ; GAUTEL M ; OESTERHELT F ; FERNANDEZ JM ; GAUB HE.** Reversible unfolding of individual titin immunoglobulin domains by AFM. *Science,* 1997, vol. 276, 1109-1112
- **SAPRA KT ; BESIR H ; OESTERHELT D ; MULLER DJ.** Mechanisms stabilizing membrane proteins assembled into oligomers. *EMBO J.,* 2005
- **WILLIAMS PM ; FOWLER SB ; BEST RB ; TOCA-HERRERA JL ; SCOTT KA ; STEWARD A ; CLARKE J.** Hidden complexity in the mechanical properties of titin. *Nature,* 2003, vol. 422, 446-449
- **ZHUANG X ; RIEF M.** Single-molecule folding. *Curr Opin Struct Biol,* 2003, vol. 13, 88-97